(19) Europäisches Patentamt
European Patent Office
Office européen des brevets

(11) Veröffentlichungsnummer: **0 031 080**
**B1**

(12) **EUROPÄISCHE PATENTSCHRIFT**

(45) Veröffentlichungstag der Patentschrift: **21.09.83**

(21) Anmeldenummer: **80107779.3**

(22) Anmeldetag: **10.12.80**

(51) Int. Cl.³: **C 07 D 409/12,** C 07 D 405/12, C 07 D 403/12, C 07 D 401/12, C 07 D 243/16, A 61 K 31/55 // (C07D409/12, 243/16, 333/38),(C07D405/12, 243/16, 307/68),(C07D403/12, 243/16, 207/34),(C07D401/12, 243/16, 213/81)

# (54) 2-Acylaminomethyl-1,4-benzodiazepine und deren Salze sowie Verfahren zu ihrer Herstellung und diese Verbindungen enthaltende Arzneimittel.

(30) Priorität: **24.12.79 DE 2952279**

(43) Veröffentlichungstag der Anmeldung: **01.07.81 Patentblatt 81/26**

(45) Bekanntmachung des Hinweises auf die Patenterteilung: **21.09.83 Patentblatt 83/38**

(84) Benannte Vertragsstaaten: **AT BE CH DE FR GB IT LI LU NL SE**

(56) Entgegenhaltungen:
**DE-A-2 353 160**
**DE-A-2 353 187**
**DE-A-2 436 147**
**US-A-3 681 340**
**US-A-4 087 421**

(73) Patentinhaber: **Kali-Chemie Pharma GmbH, Hans-Böckler-Allee 20 Postfach 220, D-3000 Hannover 1 (DE)**

(72) Erfinder: **Zeugner, Horst Dipl.-Chem. Dr.rer.nat., Havelweg 10, D-3000 Hannover 73 (DE)**
Erfinder: **Römer, Dietmar, Dr., Lettenweg 111, CH-4123 Allschwil (CH)**
Erfinder: **Liepmann, Hans, Dipl.-Chem. Dr.rer.nat., Auf dem Emmerberg 17, D-3000 Hannover 1 (DE)**
Erfinder: **Milkowski, Wolfgang, Dipl.-Chem. Dr.rer.nat., Fasanenweg 13, D-3167 Burgdorf (DE)**

(74) Vertreter: **Lauer, Dieter, Dr. et al, c/o Kali-Chemie Aktiengesellschaft Postfach 220, D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 031 080 B1

ACTORUM AG

# 2-Acylaminomethyl-1,4-benzodiazepine und deren Salze sowie Verfahren zu ihrer Herstellung und diese Verbindungen enthaltende Arzneimittel

Die vorliegende Erfindung betrifft 2-Acylaminomethyl-1,4-benzodiazepine, deren Salze sowie Verfahren zu ihrer Herstellung und diese Verbindungen enthaltende pharmazeutische Zubereitungen.

In der DE-OS 2 353 187 sind u.a. 2-Acylaminomethyl-1,4-benzodiazepine beschrieben, in welchen der Acylrest ein niedermolekulares Alkanoyl ist. Diese Substanzen besitzen vor allem eine antikonvulsive Wirkung.

Ziel der Erfindung ist es, neue 2-Acylaminomethyl-1,4-benzodiazepine mit neuartigem Wirkungsprofil zu entwickeln.

Überraschenderweise wurde dabei gefunden, dass die erfindungsgemässen 2-Acylaminomethyl-1,4-benzodiazepine neben psychopharmakologischen, diuretischen und antiarrhythmischen Wirkungen vor allem ausgeprägte analgetische Eigenschaften bei geringer Toxizität aufweisen.

Erfindungsgemäss hergestellt werden 2-Acylaminomethyl-1H-2,3-dihydro-1,4-benzodiazepine der allgemeinen Formel I

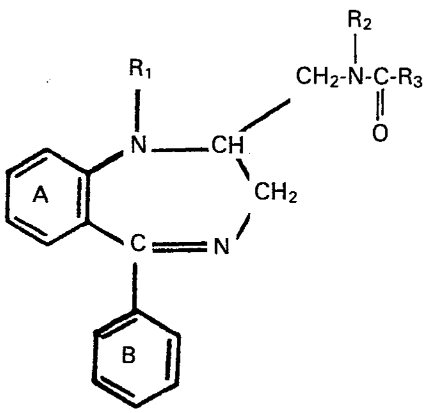

worin $R_1$ ist ein Wasserstoffatom oder eine Alkyl- oder Alkenylgruppe mit bis zu 4 Kohlenstoffatomen oder die Cyclopropylmethylgruppe,
$R_2$ ist ein Wasserstoffatom oder eine Alkyl- oder Alkenylgruppe mit bis zu 4 Kohlenstoffatomen,
$R_3$ ist eine Gruppe der Formel

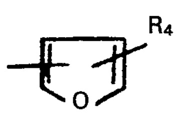
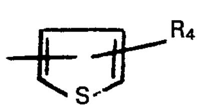
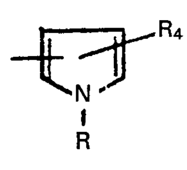

oder 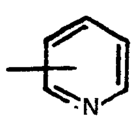

wobei R ist ein Wasserstoffatom oder ein $C_1$-$C_3$-Alkyl,
$R_4$ ist ein Wasserstoffatom, ein Halogenatom, insbesondere Chlor- oder Bromatom, eine Alkyl- oder Alkoxygruppe mit 1-4 Kohlenstoffatomen oder Nitro und
$R_4'$ ist Wasserstoff oder $C_1$-$C_4$-Alkyl,
A und B sind unabhängig voneinander unsubstituiert oder substituiert mit 1 bis 3 Substituenten, ausgewählt aus der Gruppe, bestehend aus Halogen, Alkylthio-, Alkoxy- oder Alkylgruppen mit 1-4 Kohlenstoffatomen, Hydroxy, Nitro und Trifluormethyl, oder
A und B sind Phenylreste, in welchen zwei benachbarte C-Atome durch eine Methylendioxy- oder Äthylendioxygruppe verbunden sind,
sowie deren optische Isomeren und die Säureadditionssalze der Verbindungen der allgemeinen Formel I.

Als Alkyl- und Alkenylgruppen $R_1$ und $R_2$ kommen niedermolekulare Gruppen mit bis zu 4 Kohlenstoffatomen in Frage, die gerade oder verzweigt sind, z.B. Methyl, Äthyl, Propyl, Isopropyl, n-Butyl, 1-Methylpropyl, 2-Methylpropyl, tert.-Butyl, Allyl, 2-Butenyl oder 3-Butenyl.

Für $R_4$ kommen die obengenannten niedermolekularen Alkylgruppen mit 1-4 Kohlenstoffatomen in Frage. In der niedermolekularen Alkoxygruppe kann der Alkylrest 1-4 Kohlenstoffatome haben und gerade oder verzweigt sein.

Die Alkylreste in den mit Alkyl, Alkylthio oder Alkoxy substituierten Phenylringen A und/oder B haben ebenfalls die obige Bedeutung. Insbesondere bei Di- und Trisubstitution an den Phenylringen sind der Methyl- und der Äthylrest bevorzugt. Als Halogenatome kommen Fluor, Chlor oder Brom in Frage. Für Alkylthio, Nitro und Trifluormethyl ist die Monosubstitution bevorzugt, für Halogen und/oder Alkyl bzw. und/oder Alkoxy oder Hydroxy Mono- und Disubstitution. Für niedermolekulares Alkoxy, insbesondere Methoxy, ist auch die Trisubstitution begünstigt.

Gegenstand der Erfindung ist auch ein Verfahren zur Herstellung der Verbindungen der allgemeinen Formel I. Die neuen Verbindungen werden dadurch erhalten, dass man in an sich bekannter Weise ein Amin der Formel II

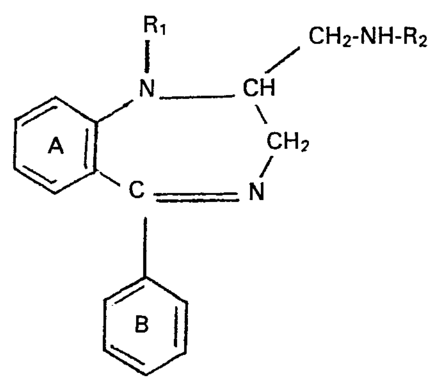

worin A, B, $R_1$ und $R_2$ die oben angegebenen Bedeutungen haben, oder deren Säureadditionssalze mit einer Carbonsäure oder einem reaktiven Carbonsäurederivat der allgemeinen Formel III

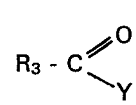

worin $R_3$ die obengenannte Bedeutung hat und Y Hydroxy, Halogen, eine niedermolekulare Alkoxygruppe oder O-CO-Z ist, worin Z die Bedeutung $R_3$ oder niedermolekulare Alkoxygruppe hat, in einem inerten Lösungsmittel bei Temperaturen zwischen $-30°C$ und dem Siedepunkt des eingesetzten Lösungsmittels umsetzt, wobei eine Verbindung der allgemeinen Formel I erhalten wird, in welcher A, B, $R_1$, $R_2$ und $R_3$ die obigen Bedeutungen haben, $R_2$ mit Bedeutung Wasserstoff ggf. in $R_2$ mit Bedeutung niedermolekulares Alkyl überführt, im Ring A in an sich bekannter Weise ggf. Chlor, Brom oder Nitro einführt, ggf. die racemischen Gemische in ihre optischen Isomeren auftrennt und ggf. die gebildete Base in ein Säureadditionssalz überführt oder aus dem Säureadditionssalz die freie Base isoliert.

Bei Verwendung eines Carbonsäurehalogenids oder Carbonsäureanhydrids wird die Umsetzung in Gegenwart eines säurebindenden Reagenzes, wie z.B. Kaliumcarbonat, Natriumcarbonat, Kaliumhydroxid, Triäthylamin, Tripropylamin, Tributylamin oder Pyridin durchgeführt. Sehr geeignet sind auch Substanzen wie 4-Dimethylaminopyridin oder 4-Pyrrolidinopyridin. Im Überschuss eingesetzt können die tertiären Amine zusätzlich als inerte Lösungsmittel dienen; dafür eignen sich aber auch beispielsweise Methylenchlorid, Chloroform, Aceton, Methylisobutylketon, Tetrahydrofuran, Dioxan, Benzol, Toluol, Xylol oder auch Chlorbenzol.

Bei Verwendung einer Verbindung III, in welcher Y die Bedeutung einer niedermolekularen Alkoxygruppe hat, wird die Umsetzung zweckmässigerweise in einem geschlossenen Gefäss durchgeführt, wobei auch im Überschuss eingesetzter Ester als Lösungsmittel dienen kann. Die Umsetzung kann durch Zugabe eines Metallalkoholats katalysiert werden, beispielsweise durch Zusatz von Aluminiumisopropylat.

Bei Verwendung einer Verbindung III, in welcher Y die Bedeutung Halogen hat, eignen sich insbesondere solche Verbindungen mit Y gleich Chlor.

Die neuen Verbindungen der allgemeinen Formel I lassen sich auch dadurch erhalten, dass man eine Verbindung der allgemeinen Formel II, in der A und B, $R_1$ und $R_2$ die oben angegebenen Bedeutungen haben, in einem inerten Lösungsmittel bei Temperaturen von $-30°C$ zur Siedetemperatur des Lösungsmittels mit einer Carbonsäure der allgemeinen Formel IV,

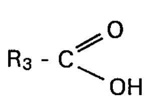

wobei $R_3$ die obengenannte Bedeutung hat, in Gegenwart eines geeigneten Kopplungsreagenzes, wie beispielsweise Dicyclohexylcarbodiimid, Carbonyldiimidazol oder dergleichen, umsetzt. Vorzugsweise wird die Umsetzung bei Temperaturen von $-30$ bis $+30°C$ in Lösungsmitteln, wie Methylenchlorid, Chloroform, Benzol oder Toluol durchgeführt. Die hierbei erhaltenen Verbindungen der allgemeinen Formel I, in der $R_2$ die Bedeutung Wasserstoff hat, können durch nachträgliche Alkylierung in an sich bekannter Weise in die entsprechenden N-Alkylverbindungen übergeführt werden. Dies kann beispielsweise dadurch bewirkt werden, dass man in einer Verbindung der allgemeinen Formel I mit $R_2$ gleich Wasserstoff diesen unter Verwendung eines geeigneten, inerten Lösungsmittels durch Umsetzung mit einem Metallierungsmittel durch Metall ersetzt und anschliessend diese metallierte Verbindung mit einem Alkylhalogenid, Alkylsulfat oder Alkylsulfonsäureester umsetzt.

Die Metallierungsreaktion wie die Alkylierung können bei Temperaturen von $-80°C$ bis zur Siedetemperatur des verwendeten Lösungsmittels durchgeführt werden.

Als Metallierungsmittel eignen sich beispielsweise Natriumhydrid, Lithiumbutyl, Lithiumphenyl, Natriumamid, Lithiumdiisopropylamid, aber auch Natriumalkoxid und Thallium-I-alkoxid.

Inerte Lösungsmittel sind in Abhängigkeit vom verwendeten Metallierungsmittel Diäthyläther, Tetrahydrofuran, Dioxan, Benzol, Toluol, Dimethylformamid, Dimethylsulfoxid, für die Metallalkoxide aber auch die entsprechenden Alkohole, also für Methoxide Methanol und für Äthoxide Äthanol.

Die erfindungsgemässen Verbindungen der allgemeinen Formel I werden bei der Synthese in Form ihrer Racemate erhalten. Unter den Schutz dieser Erfindung fallen nun sowohl die racemischen Gemische wie auch die optisch aktiven Formen. Die optisch aktiven Verbindungen können aus den racemischen Gemischen der allgemeinen Formel I in an sich bekannter Weise mit geeigneten optisch aktiven Säuren, wie beispielsweise Weinsäure, O,O'-Dibenzoylweinsäure, Mandelsäure, Di-O-isopropyliden-2-oxo-L-gulonsäure und anschliessende fraktionierte Kristallisation der gewonnenen Salze in ihre optisch aktiven Antipoden aufgetrennt werden [S.H. Willen, A. Collet, J. Jacques, Tetrahedron *33,* (1977) 2725-2736]. Aus diesen Salzen kann man die freien Basen gewinnen, und diese gewünschtenfalls in pharmakologisch verträgliche Salze überführen. Durch Umkristallisation aus Lösungsmitteln, wie niederen Alkoholen und/oder Äther, können die racemischen Gemische und ihre optisch aktiven Isomeren sowie deren Säureadditionssalze gereinigt werden.

Die Auftrennung in die optisch aktiven Verbindungen kann aber auch in einer geeigneten Vorstufe erfolgen.

Die Herstellung der als Ausgangssubstanzen verwendeten 2-Aminomethyl-1H-2,3-dihydro-1,4-benzodiazepine der allgemeinen Formel II kann in an sich bekannter Weise nach Verfahren erfolgen, die in der DE-OS 2 221 558 beschrieben sind. Ausgangsprodukte für die Verbindungen der Formel II sind danach 1-Acyl-2-hydroxy-1,3-diaminopropane, deren Herstellung in den deutschen Offenlegungsschriften 2 221 558, 2 314 993, 2 720 915 und 2 720 968 beschrieben ist.

Danach kann man 2-Hydroxy-1,3-diaminopropane der allgemeinen Formel V,

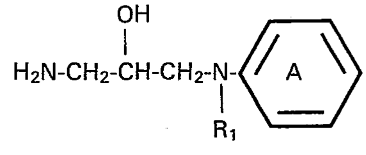

in der $R_1$ und A die oben angegebenen Bedeutungen haben, mit einem gegebenenfalls substituierten Benzoylchlorid zu Verbindungen der allgemeinen Formel VI,

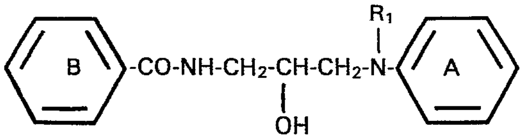

in der A und B und $R_1$ die oben angegebenen Bedeutungen haben, umsetzen.

Die Ausgangsverbindungen der allgemeinen Formel V können auf die von M. Chadwick et al. in J. Med. Chem. *9,* S. 874 (1966) beschriebene Weise hergestellt werden.

Die Verbindungen der allgemeinen Formel VI, in denen $R_1$ die Bedeutung Wasserstoff hat, können auf dieser Stufe durch nachträgliche Alkylierung in an sich bekannter Weise in die entsprechenden N-Alkylverbindungen übergeführt werden. Dies geschieht beispielsweise nach den aus der Literatur bekannten Verfahren der reduktiven Carbonyl-Aminierung wie der Leuckart-Wallach- bzw. Eschweiler-Clarke Reaktion [s. H. Krauch, W. Kunz, Reaktionen der Organischen Chemie (1976), S. 126 und 131] oder durch Alkylierung mit Dialkylsulfaten [s. Houben-Weyl XI/1 (1957), S. 207 ff.].

Die auf die vorstehende Weise erhaltenen Verbindungen der allgemeinen Formel VI können nun in an sich bekannter Weise, wie bereits in den deutschen Offenlegungsschriften 2 221 558, 2 314 993 und 2 520 937 beschrieben, durch Umsetzung mit Phosphoroxidhalogeniden, vorzugsweise Phosphoroxidchlorid, cyclisiert werden. Man behandelt dazu zweckmässigerweise Verbindungen der allgemeinen Formel VI oder deren Säureadditionssalze, wie in DE-OS 2 520 937 beschrieben, mit dem Cyclisierungsmittel bei einer Temperatur zwischen 100 und 150°C und isoliert anschliessend das Gemisch der beiden isomeren Verbindungen mit den allgemeinen Formeln VII und VIII,

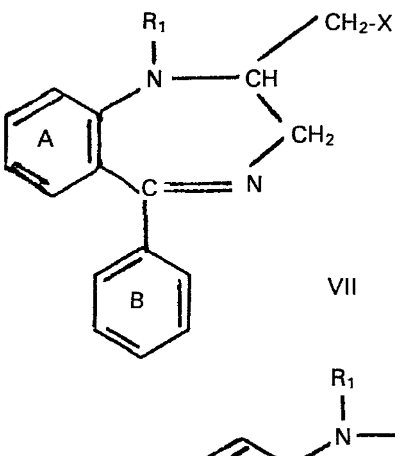

VII

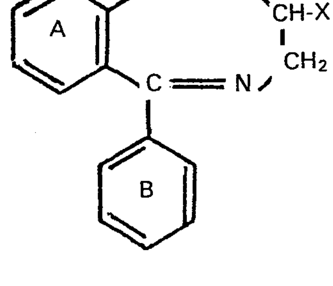

VIII

wobei A und B und $R_1$ die oben angegebenen Bedeutungen haben und X Halogen, vorzugsweise Chlor ist.

Die beiden isomeren Verbindungen VII und VIII liegen im Reaktionsgemisch in wechselnden Mengenverhältnissen von VII zu VIII vor in Abhängigkeit von der Art der Substituenten an den Phenylringen A und B, sowie von deren Stellung. Dies ist jedoch für die folgende Umsetzung dieses Gemisches ohne Belang, da beide Isomere in einheitlicher Reaktion zu Verbindungen der allgemeinen Formel II, in der A, B, $R_1$ und $R_2$ die oben angegebenen Bedeutungen haben, reagieren [s. dazu auch Milkowski et al. Eur. J. Med. Chem. *6,* S. 501-507 (1976)]. Eine langwierige Trennung oder Analyse des Isomerengemisches ist daher nicht nötig.

So kann man das, wie oben beschrieben erhaltene Isomerengemisch von Verbindungen der allgemeinen Formeln VII und VIII nach grober Abtrennung von Nebenprodukten, jedoch ohne Trennung in die Einzelkomponenten, wie in der DOS 2 353 187 für die Verbindungen des Typs VII beschrieben, mit Alkalimetallimiden, vorzugsweise mit Kaliumphthalimid, zu 2-Phthalimidoverbindungen der allgemeinen Formel IX

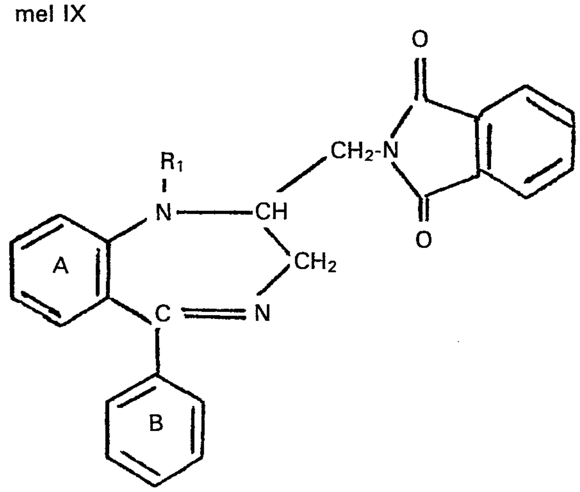

wobei A und B und $R_1$ die oben angegebenen Bedeutungen haben, umsetzen und die so erhaltenen Verbindungen der allgemeinen Formel IX roh oder nach vorhergehender Reinigung in an sich bekannter Weise mit Hydrazinhydrat oder mit verdünnter Salzsäure zu Verbindungen der allgemeinen Formel II, wobei A und B und $R_1$ die oben angegebenen Bedeutungen haben und $R_2$ gleich Wasserstoff ist, spalten [s. H. Krauch, W. Kunz, Reaktionen der Organischen Chemie (1976), S. 638]. Zur Herstellung der 2-Phthalimidoverbindungen der allgemeinen Formel IX werden zweckmässig Lösungsmittel wie Methanol, Äthanol, Isopropaol, Dioxan, Dimethylformamid mit oder ohne Zusatz von Kaliumjodid als Katalysator und Temperaturen von 50 bis 130°C angewendet.

Für die Spaltungsreaktion von Verbindungen der allgemeinen Formel IX zu Verbindungen der allgemeinen Formel II mit $R_2$ gleich Wasserstoff werden vorzugsweise Alkohole wie Methanol, Äthanol, Isopropanol, t-Butanol oder Wasser und Temperaturen von 20 bis 120°C, zweckmässig jedoch die Siedetemperatur des verwendeten Lösungsmittels, angewendet.

Man kann das Isomerengemisch der allgemeinen Formeln VII und VIII auch dadurch in Verbindungen der allgemeinen Formel II umwandeln, in der $R_2$ ausser Wasserstoff auch die anderen oben für $R_2$ angegebenen Bedeutungen haben kann, dass man das erwähnte Gemisch, wie in der DOS 2 221 558 für Verbindungen des Typs VII beschrieben, für $R_2$ gleich Wasserstoff mit Ammoniak oder für $R_2$ ungleich Wasserstoff mit geeigneten primären Aminen, wie beispielsweise Methylamin, Äthylamin, Propylamin, Butylamin, Allylamin, Cyclopropylmethylamin, mit oder ohne Lösungsmittel, bei Normaldruck oder bei erhöhtem Druck bei Temperaturen von 20 bis 150°C umsetzt. Zweckmässig kann bei derartigen Umsetzungen überschüssiges Amin als Lösungsmittel dienen, man kann aber auch inerte Lösungsmittel wie Wasser, Methanol, Äthanol, Isopropanol, t-Butanol, Dioxan, Benzol, Toluol oder Xylol verwenden.

Eine Variante dieses Verfahrens besteht darin, dass man anstelle von Ammoniak oder der primären Amine deren Alkalimetallsalze mit dem Isomerengemisch von Verbindungen der allgemeinen Formeln VII und VIII reagieren lässt. Als Metalle kommen vor allen Dingen Lithium und Natrium in Frage, als inerte Lösungsmittel dienen zweckmässigerweise überschüssiges Ammoniak oder die entsprechenden primären Amine, man kann aber auch inerte Lösungsmittel wie Tetrahydrofuran, Dioxan, Benzol und Toluol verwenden. Die Alkalimetallsalze können in situ erzeugt oder in fester Form zugesetzt werden, die Temperaturen für die Umsetzungen liegen zwischen −50 und 150°C.

Nach einem neuen Verfahrensaspekt zur Herstellung der erfindungsgemässen Verbindungen der Formel I, in der A, B, $R_1$ und $R_2$ die oben angegebenen Bedeutungen haben, werden durch Umsetzung der Verbindungen der allgemeinen Formeln VII und/oder VIII mit einem Alkalimetallazid, vorzugsweise Natrium- oder Kaliumazid, in einem inerten Lösungsmittel bei Temperaturen von −30 bis 150°C die 2-Azidomethyl-1,4-benzodiazepine der allgemeinen Formel X

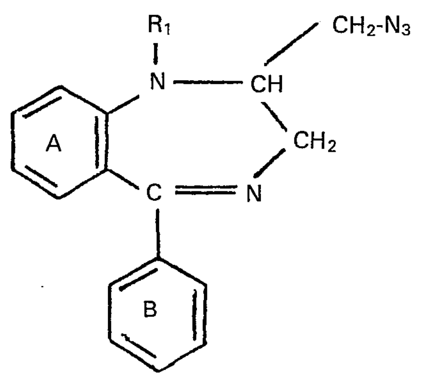

worin A und B und $R_1$ die oben angegebenen Bedeutungen haben, hergestellt. Diese Verbindungen können in Form ihrer Basen oder Säureadditionssalze isoliert werden, sie können aber auch, ggf. nach Entfernung des Lösungsmittels, ohne Abtrennung aus dem Reaktionsgemisch, weiter umgesetzt werden.

Geeignete Lösungsmittel für die Herstellung der Azide der Formel X sind beispielsweise Methylenchlorid, Chloroform, Tetrahydrofuran, Dioxan, Dimethylformamid, Dimethylsulfoxid, Hexamethylphosphortriamid, Methanol, Äthanol, t-Butanol, Aceton, Methylisobutylketon.

Durch anschliessende Spaltung dieser Verbindungen in an sich bekannter Weise mit Hydrazinhydrat/Raney-Nickel unter basischer Katalyse oder mit Propandithiol erhält man die 2-Aminomethyl-1,4-benzodiazepine der allgemeinen Formel II mit $R_2$ gleich Wasserstoff. Die Reduktion mit Hydrazin erfolgt zweckmässigerweise in Alkoholen wie Methanol, Äthanol unter Zusatz von tertiären Aminen wie Triäthylamin bei Raumtemperatur. Bei der Reduktion mit Propandithiol dienen Methanol, Äthanol, Dimethylformamid oder Pyridin/Wasser als Lösungsmittel.

Auf die oben beschriebene Weise können 2-Aminomethylverbindungen in die gewünschten 2-Acylaminomethyl-1,4-benzodiazepine der allgemeinen Formel I übergeführt werden.

Ausdrücklich soll an dieser Stelle betont werden, dass für die oben angegebenen Reaktionen zur Herstellung der Verbindungen der allgemeinen Formeln II, IX und X zwar zweckmässigerweise das Isomerengemisch der Verbindungen VII und VIII verwendet werden kann, doch ist es für den Fachmann offensichtlich, dass man das Isomerengemisch auch in die Einzelkomponenten trennen und diese dann separat in den angegebenen Reaktionen zu Verbindungen der allgemeinen Formeln II, IX und X umsetzen kann.

Eine nachträgliche Substitution des 1,4-Benzodiazepinsystems ist im Phenylring A in an sich bekannter Weise durch Halogen oder die Nitrogruppe möglich, wie in der DE-OS 2 221 558 bereits beschrieben. Zweckmässigerweise bieten sich als geeignete Produkte für eine solche Substitution Verbindungen der allgemeinen Formeln I, VII, IX und X an. Als Halogenierungsmittel können beispielsweise N-Chlorsuccinimid oder N-Bromsuccinimid dienen. Zur Einführung der Nitrogruppe können die üblichen Nitrierungsreagenzien herangezogen werden, beispielsweise $KNO_3$ in $H_2SO_4$ oder Kupfer-II-nitrat-trihydrat in Acetanhydrid.

Man kann auch die als Ausgangssubstanzen verwendeten Verbindungen der allgemeinen Formel II, worin A und B mit Ausnahme von Alkyloxy und Alkylthio die oben angegebenen Bedeutungen haben und $R_1$ gleich Wasserstoff ist, auch dadurch erhalten, dass man Verbindungen der allgemeinen Formel II mit $R_1$ gleich Alkyl, vorzugsweise Methyl mit Jodwasserstoffsäure entalkyliert. Die Reaktion wird in konzentrierter Jodwasserstoffsäure bei Temperaturen zwischen 50 und 100°C durchgeführt.

Die nach den erfindungsgemässen Verfahren erhaltenen Verbindungen der allgemeinen Formel I werden entweder als freie Basen isoliert oder gewünschtenfalls in üblicher Weise in ihre Additionssalze mit anorganischen oder organischen Säuren übergeführt. Dazu versetzt man beispielsweise eine Lösung einer Verbindung der allgemeinen Formel I in einem geeigneten Lösungsmittel mit der als Salzkomponente gewünschten Säure. Vorzugsweise wählt man für die Umsetzung organische Lösungsmittel, in denen das enstehende Salz unlöslich ist, damit es durch Filtration abgetrennt werden kann. Solche Lösungsmittel sind beispielsweise Äthanol, Isopropanol, Äther, Aceton, Essigsäureäthylester, Aceton-Äther, Aceton-Äthanol, Äthanol-Äther.

Aus dem US-Patent Nr. 3 681 340 sind 1,3-Benzodiazepin-Derivate bekannt, welche durch Herz- und Kreislaufwirkungen, insbesondere koronare Gefässerweiterung und Erhöhung der Kontraktionskraft, ausgezeichnet sind und daneben auch in geringerem Masse analgetische Eigenschaften zeigen. Das US-Patent Nr. 4 087 421 beschreibt tricyclische vom 1,5-Benzodiazepin-Gerüst abgeleitete Thieno[3,4-b][1,5]-benzodiazepine mit neuroleptischen, antidepressiven und analgetischen Eigenschaften. In keiner der in den genannten US-Patenten beschriebenen Substanzgruppen ist jedoch die analgetische Wirkungskomponente vorherrschend.

Aus der Literatur ist nun bekannt, dass in 2-Stellung substituierte 1,4-Benzodiazepine wertvolle pharmakologische Eigenschaften bei niedriger Toxizität aufweisen. Insbesondere beeinflussen diese beschriebenen Verbindungen das Zentralnervensystem in spezifischer Weise (s. DE-OS 2 520 937 und 2 754 112). Die Wirkungen dieser Substanzen sind derart, dass sie vor allen Dingen aufgrund ihrer anxiolytischen und aggressionsdämpfenden Eigenschaften wertvolle Arzneimittel zur Behandlung dieser Symptomatik beim Menschen darstellen. Es war nun um so überraschender, dass die neuen erfindungsgemässen 2-Acylaminomethyl-1,4-benzodiazepine ein pharmakologisch neuartiges Wirkungsprofil zeigen, wobei neben psychopharmakologischen, diuretischen und antiarrhythmischen Wirkungen ausgeprägte analgetische Eigenschaften bei geringer Toxizität in verschiedenen Tests an kleinen Nagern und an Affen auffielen.

Die erfindungsgemässen Verbindungen sind nützliche Analgetika, nachweisbar durch ihre Eigenschaften, die Schmerzschwelle bei Säugetieren heraufzusetzen. Dies wurde untersucht in zwei pharmakologischen Standard-Methoden, dem Brennstrahltest an der Maus und dem Arthritisschmerztest an der Ratte.

*Beschreibung der pharmakologischen Untersuchungsmethoden*

*1. Bestimmung der minimalen toxischen Dosis*

Drei männlichen Mäusen mit 20 bis 25 g Gewicht werden per os Maximaldosen von 300 mg/kg der Testsubstanz verabreicht und drei Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Die Testsubstanzen werden je nach Wasserlöslichkeit entweder als wässrige Lösungen oder als Suspensionen ohne Verwendung von Lösungsmitteln hergestellt. Um die Testsubstanz in einer stabilen Suspension zu halten, kann ein Tropfen Tween® -80 zugesetzt und das Ganze mechanisch homogenisiert werden. Wenn Tod oder toxische Symptome beobachtet werden, werden weiteren Mäusen zunehmend geringere Dosen verabreicht, bis keine toxischen Symptome mehr auftreten. Die niedrigste Dosis, welche toxische Symptome hervorruft, wird als minimale toxische Dosis angegeben.

*2. Arthritisschmerztest an der Ratte*

Männlichen Ratten vom Stamm OFA mit einem Gewicht von 160-180g werden mit 20 mg/kg i.p. Pentobarbitalnatrium anästhesiert und 0,1 ml einer Suspension von Mycobacterium smegmae (SI 043) in Paraffinöl (0,6 mg Mycobact./0,1 ml Öl) intrakutan indie linke Hinterpfote injiziert. 14 Tage später, wenn sich besonders in der rechten Hinterpfote eine ausgeprägte sekundäre Arthritis entwickelt hat, werden die Wirkungen der Testsubstanzen untersucht. 30 Minuten vor Verabreichung der Testsubstanzen wird eine Kontrollmessung vorgenommen, indem das Fussgelenk der rechten Hinterpfote dreimal gebeugt wird und die Anzahl der Lautgebungen gezählt werden. Ratten, welche nicht reagieren, werden ausgesondert. 3 Stunden nach oraler Gabe der Testsubstanzen wird die Beugeprozedur wiederholt. Tiere, welche entweder nur einmal oder überhaupt nicht Laut geben, werden als geschützt gegen Schmerzen angesehen. Zwischen 9 und 20 Ratten werden pro Dosis verwendet und die $ED_{50}$ (95% Vertrauensbereich), bestimmt nach der Methode von Litchfield und Wilcoxon (1949), wird als Dosis bezeichnet, welche einen Schutz in 50% der behandelten Tiere erzeugt.

*3. Brennstrahl-Test an der Maus*

Die Methode basiert auf der von D'Amour und Smith beschriebenen Arbeitsweise (1941), es werden aber gefütterte männliche und weibliche Mäuse mit 16-25 g Körpergewicht anstelle von Ratten verwendet. 30 Minuten vor der Behandlung mit der Testsubstanz wurde jede Maus einzeln in ein zylindrisches Gefäss gesetzt, so dass sie sich nicht umdrehen und vorwärt bewegen konnte. Ihr Schwanz ragte in einer engen Rinne liegend aus dem Gefäss heraus. Ein bestimmter Punkt des Schwanzes jedes Tieres (ungefähr 35 mm von der Schwanzwurzel entfernt) wurde der Bestrahlungshitze einer Lampe bekannter Stärke und Temperatur ausgesetzt, die sich direkt unter dem Schwanz befand. Die Zeit in Sekunden, die die Maus brauchte, um den Schwanz aus dem Lichtstrahl zu schnellen, wurde zweimal bestimmt, einmal 30 und einmal 15 Minuten vor subkutaner Verabreichung der Testsubstanz (10 mg/kg). Die Mäuse, deren Reaktionszeiten um mehr als 25% abwichen, wurden ausgeschieden. Die Reaktionszeiten wurden erneut nach 15 Minuten und 30 Minuten nach der Behandlung gemessen und eine Ausdehnung der Reaktionszeiten auf mehr als 75% der durchschnittlichen Vorbehandlungswerte derselben Maus als analgetischer Effekt angesehen. Als $ED_{50}$ (95% Vertrauensbereich) jeder Testsubstanz 30 Minuten nach Verabreichung, bewertet nach Litchfield und Wilcoxon (1949), wurde die Dosis angesehen, welche die Vorbehandlungsreaktionszeit um mehr als 75% in 50% der Tiere verlängerte.

Die nachfolgenden Verbindungen wurden nach den vorstehenden Testmethoden geprüft:

1) 1-Methyl-2-[(thiophen-2-carbonyl)-aminomethyl]-5-phenyl-1H-2,3-dihydro-1,4-benzodiazepin (Sesquitartrat/Hemiisopropylat)
2) 1-Methyl-2-[(furan-2-carbonyl)-aminomethyl]-5-phenyl-1H-2,3-dihydro-1,4-benzodiazepin (Hydrochlorid/Semihydrat)

3) 8-Methoxy-1-methyl-2-[(thiophen-2-carbonyl)-aminomethyl]-5-phenyl-1H-2,3-dihydro-1,4-benzodiazepin (Hydrochlorid)
4) 8-Methoxy-1-methyl-2-[(furan-2-carbonyl)-aminomethyl]-5-phenyl-1H-2,3-dihydro-1,4-benzodiazepin (Hydrochlorid)
5) 8-Methoxy-1-methyl-2-[(thiophen-3-carbonyl)-aminomethyl]-5-phenyl-1H-2,3-dihydro-1,4-benzodiazepin (Hydrochlorid)
6) 8-Methoxy-1-methyl-2-[(furan-3-carbonyl)-aminomethyl]-5-phenyl-1H-2,3-dihydro-1,4-benzodiazepin (Hydrochlorid)
7) 1-Methyl-2-[(thiophen-3-carbonyl)-aminomethyl]-5-phenyl-1H-2,3-dihydro-1,4-benzodiazepin (Hydrochlorid)
8) 1,7,8-Trimethyl-2-[(furan-3-carbonyl)-aminomethyl]-5-phenyl-1H-2,3-dihydro-1,4-benzodiazepin (Hydrochlorid)
9) 8-Methoxy-1-methyl-2-[(5-methylthiophen-2-carbonyl)-aminomethyl]-5-phenyl-1H-2,3-dihydro-1,4-benzodiazepin (Hydrochlorid)
10) 1,7-Dimethyl-2-[(thiophen-3-carbonyl)-aminomethyl]-5-phenyl-1H-2,3-dihydro-1,4-benzodiazepin (Dihydrochlorid)
11) 1,7-Dimethyl-2-[(furan-3-carbonyl)-aminomethyl]-5-phenyl-1H-2,3-dihydro-1,4-benzodiazepin (Hydrochlorid)
12) 1-Methyl-2-[(5-methylthiophen-2-carbonyl)-aminomethyl]-5-phenyl-1H-2,3-dihydro-1,4-benzodiazepin (Hydrochlorid; 0,4 Aceton)
13) 8-Äthoxy-1-methyl-2-[(furan-3-carbonyl)-aminomethyl]-5-phenyl-1H-2,3-dihydro-1,4-benzodiazepin (Hydrochlorid; 0,5 Wasser, 0,5 Aceton)
14) 1-Methyl-2-[(furan-2-carbonyl)-aminomethyl]-5-(3-methoxyphenyl)-1H-2,3-dihydro-1,4-benzodiazepin (Hydrochlorid)
15) 7-Methoxy-1-methyl-2-[(furan-3-carbonyl)-aminomethyl]-5-phenyl-1H-2,3-dihydro-1,4-benzodiazepin (Sesquihydrochlorid; 0,3 Wasser)
16) 1-Methyl-2-[(thiophen-3-carbonyl)-aminomethyl]-5-(3-methoxyphenyl)-1H-2,3-dihydro-1,4-benzodiazepin (Hydrochlorid)
17) 1-Äthyl-2-[(furan-3-carbonyl)-aminomethyl]-5-phenyl-1H-2,3-dihydro-1,4-benzodiazepin (Hydrochlorid; 0,15 Wasser)
18) 1-Methyl-2-[(furan-2-carbonyl)-aminomethyl]-5-(2-fluorphenyl)-1H-2,3-dihydro-1,4-benzodiazepin (Hydrochlorid)
19) 1-Methyl-2-[(furan-3-carbonyl)-aminomethyl]-5-(2-fluorphenyl-1H-2,3-dihydro-1,4-benzodiazepin (Hydrochlorid)
20) 1-Methyl-2-[(thiophen-3-carbonyl)-aminomethyl]-5-(2-fluorphenyl)-1H-2,3-dihydro-1,4-benzodiazepin (Hydrochlorid)
21) 1-Methyl-2-[(thiophen-2-carbonyl)-aminomethyl]-5-(2-fluorphenyl)-1H-2,3-dihydro-1,4-benzodiazepin (Hydrochlorid)
22) 1-Methyl-2-[(furan-3-carbonyl)-aminomethyl]-5-phenyl-1H-2,3-dihydro-1,4-benzodiazepin
23) 8-Methoxy-1-methyl-2-[(3-methylthiophen-2-carbonyl)-aminomethyl]-5-phenyl-1H-2,3-dihydro-1,4-benzodiazepin (Hydrochlorid)
24) 7,8-Methylendioxy-1-methyl-2-[(thiophen-2-carbonyl)-aminomethyl]-5-phenyl-1H-2,3-dihydro-1,4-benzodiazepin (Hydrochlorid)
25) 7,8-Methylendioxy-1-methyl-2-[(furan-2-carbonyl)-aminomethyl]-5-phenyl-1H-2,3-dihydro-1,4-benzodiazepin (Hydrochlorid)
26) 7,8-Methylendioxy-1-methyl-2-[(furan-3-carbonyl)-aminomethyl]-5-phenyl-1H-2,3-dihydro-1,4-benzodiazepin
27) 8-Methoxy-1-methyl-2-[(5-bromthiophen-2-carbonyl)-aminomethyl]-5-phenyl-1H-2,3-dihydro-1,4-benzodiazepin (Hydrochlorid)
28) 2-[(Thiophen-2-carbonyl)-aminomethyl]-5-phenyl-1H-2,3-dihydro-1,4-benzodiazepin
29) 8-Methoxy-1-methyl-2-nicotinoylaminomethyl-5-phenyl-1H-2,3-dihydro-1,4-benzodiazepin (Dihydrochlorid; 0,4 Wasser; 0,8 Aceton)
30) 8-Methoxy-1-methyl-2-picolinoylaminomethyl-5-phenyl-1H-2,3-dihydro-1,4-benzodiazepin (1,8 Hydrochlorid)
31) 8-Methoxy-1-methyl-2-[(5-methylfuran-2-carbonyl)-aminomethyl]-5-phenyl-1H-2,3-dihydro-1,4-benzodiazepin (Hydrochlorid)
32) 7,8-Methylendioxy-1-methyl-2-[(5-methylthiophen-2-carbonyl)-aminomethyl]-5-phenyl-1H-2,3-dihydro-1,4-benzodiazepin (Hydrochlorid)
33) 8-Methoxy-1-methyl-2-[(1-methylpyrrol-2-carbonyl)-aminomethyl]-5-phenyl-1H-2,3-dihydro-1,4-benzodiazepin (Hydrochlorid)
34) 8-Methoxy-1-methyl-2-[(furan-3-carbonyl)-aminomethyl]-5-(4-fluorphenyl)-1H-2,3-dihydro-1,4-benzodiazepin (Hydrochlorid)
35) 8-Methoxy-1-methyl-2-[(furan-3-carbonyl)-aminomethyl]-5-(2-trifluormethylphenyl)-1H-2,3-dihydro-1,4-benzodiazepin (Hydrochlorid)
36) 1-Methyl-2-[(1-methylpyrrol-2-carbonyl)-aminomethyl]-5-phenyl-1H-2,3-dihydro-1,4-benzodiazepin (Hydrochlorid)
37) 8-Chlor-1-methyl-2-[(furan-3-carbonyl)-aminomethyl]-5-phenyl-1H-2,3-dihydro-1,4-benzodiazepin (Hydrochlorid)
38) 8-Fluor-1-methyl-2-[(furan-3-carbonyl)-aminomethyl]-5-phenyl-1H-2,3-dihydro-1,4-benzodiazepin (Hydrochlorid)
39) 8-Methoxy-1-methyl-2-[(furan-3-carbonyl)-aminomethyl]-5-(2-fluorphenyl)-1H-2,3-dihydro-1,4-benzodiazepin (Hydrochlorid)
40) 8-Methoxy-1-methyl-2-[(thiophen-2-carbonyl)-aminomethyl]-5-(2-fluorphenyl)-1H-2,3-dihydro-1,4-benzodiazepin (Hydrochlorid)
41) 7,8-Methylendioxy-1-methyl-2-[(thiophen-3-carbonyl)-aminomethyl]-5-phenyl-1H-2,3-dihydro-1,4-benzodiazepin (Hydrochlorid)
42) 8-Methoxy-1-methyl-2-[(furan-3-carbonyl)-aminomethyl]-5-(3-fluorphenyl)-1H-2,3-dihydro-1,4-benzodiazepin (Hydrochlorid)
43) 8-Fluor-1-methyl-2-[(5-methylthiophen-2-carbonyl)-aminomethyl]-5-phenyl-1H-2,3-dihydro-1,4-benzodiazepin (Hydrochlorid)
44) 7,8-Äthylendioxy-1-methyl-2-[(furan-3-carbonyl)-aminomethyl]-5-phenyl-1H-2,3-dihydro-1,4-benzodiazepin (Hydrochlorid)
45) 1,7-Dimethyl-2-[(furan-3-carbonyl)-aminomethyl]-5-(2-fluorphenyl)-1H-2,3-dihydro-1,4-benzodiazepin (1,4 Hydrochlorid; 0,5 Wasser; 0,2 Äthylacetat)
46) 8-Methoxy-1-methyl-2-[(5-bromthiophen-2-carbonyl)-aminomethyl]-5-(2-fluorphenyl)-1H-

-2,3-dihydro-1,4-benzodiazepin (Hydrochlorid)

47) 8-Methoxy-1-methyl-2-[(thiophen-2-carbonyl)-aminomethyl]-5-(4-fluorphenyl)-1H-2,3-dihydro-1,4-benzodiazepin (Hydrochlorid)

48) 8-Methoxy-1-methyl-2-[(5-bromthiophen-2-carbonyl)-aminomethyl]-5-(3-fluorphenyl)-1H-2,3-dihydro-1,4-benzodiazepin (Hydrochlorid)

49) 8-Methoxy-1-methyl-2-[(5-bromthiophen-2-carbonyl)-aminomethyl]-5-(4-fluorphenyl)-1H-2,3-dihydro-1,4-benzodiazepin (Hydrochlorid)

Die in der Tabelle aufgezeigten pharmakologischen Ergebnisse zeigen, dass die erfindungsgemässen Substanzen die Schmerzschwelle bei Säugetieren und beim Menschen erheblich heraufsetzen. Sie stellen somit wertvolle Analgetika zur Behandlung von Schmerzen aller Art in Dosierungen von 0,1 bis 100 mg/kg dar.

Tabelle

| Substanz Nr. | MDL (Maus) p.o. mg/kg | Brennstrahltest (Maus) s.c. $ED_{50}$ mg/kg | Arthritisschmerztest (Ratte) p.o. $ED_{50}$ mg/kg |
|---|---|---|---|
| 1 | >300 | 2,8 | 13 |
| 2 | >300 | 2,8 | 32 |
| 3 | >200 | 5,3 | 20 |
| 4 | >200 | 7,0 | 21 |
| 5 | >200 | 2,4 | 15 |
| 6 | > 50 | 2,8 | 9 |
| 7 | >200 | 0,22 | 19 |
| 8 | >200 | 8,0 | 14 |
| 9 | >200 | 5,6 | 14 |
| 10 | >300 | 3,2 | 32 |
| 11 | >300 | 1,2 | 30 |
| 12 | >300 | 2,8 | 10 |
| 13 | >200 | 6,0 | 15 |
| 14 | >300 | 1,6 | 32 |
| 15 | >300 | 8,5 | 32 |
| 16 | >300 | 0,5 | 32 |
| 17 | >200 | 2,6 | 22 |
| 18 | >300 | 4,1 | 18 |
| 19 | >300 | 0,56 | 13 |
| 20 | >300 | 1,1 | 18 |
| 21 | >300 | 6,2 | 13 |
| 22 | | 0,44 | 14 |
| 23 | | 7,2 | 25 |
| 24 | | 3,8 | 10 |
| 25 | | <5,6 | <32 |
| 26 | | 2,0 | < 5,6 |
| 27 | | >5,6 | <32 |
| 28 | | 4,2 | >32 |
| 29 | | 4,0 | 30 |
| 30 | | 7,0 | >32 |
| 31 | | 4,4 | 30 |
| 32 | | 5,6 | <18 |
| 33 | | 1,0 | <18 |
| 34 | | 2,1 | 5,6 |
| 35 | | 1,0 | <18 |
| 36 | | 2,1 | >18 |
| 37 | | 1,2 | >18 |
| 38 | | 0,32 | <18 |
| 39 | | 0,7 | <10 |
| 40 | | 1,2 | <18 |
| 41 | | 2,1 | 5,6 |
| 42 | | 5,6 | <18 |
| 43 | | 5,6 | <18 |
| 44 | | >5,6 | <18 |
| 45 | | 3,8 | <18 |
| 46 | | 5,6 | <18 |
| 47 | | 3,2 | <18 |
| 48 | | >5,6 | <18 |
| 49 | | >5,6 | <18 |

Zur Verwendung als Arzneistoffe können sowohl die freien Basen als auch deren pharmazeutisch annehmbare Säureadditionssalze eingesetzt werden, d.h. Salze mit solchen Säuren, deren Anionen bei den in Frage kommenden Dosierungen nicht toxisch sind. Ferner ist es von Vorteil, wenn die als Arzneistoffe zu verwendenden Salze gut kristallisierbar und nicht oder nur wenig hygroskopisch sind. Zur Salzbildung mit Verbindungen der allgemeinen Formel I eignen sich beispielsweise für diese Zwecke Chlorwasserstoffsäure, Bromwasserstoffsäure, Schwefelsäure, Phosphorsäure, Methansulfonsäure, Äthansulfonsäure, Benzolsulfonsäure, p-Toluolsulfonsäure, Citronensäure, Essigsäure, Milchsäure, Bernsteinsäure, Maleinsäure, Fumarsäure, Äpfelsäure, Weinsäure, Benzoesäure, Phenylessigsäure und Mandelsäure.

Die Verbindungen der Formel I können in pharmazeutischen Gebrauchsformen verabreicht werden, welche etwa 0,1 bis 100 mg Aktivsubstanz enthalten, wobei die Dosierung der zu behandelnden Spezies und den individuellen Erfordernissen angepasst wird. Parenterale Formulierungen werden im allgemeinen weniger Aktivsubstanz als Präparate zu oraler Verabreichung enthalten. Die Verbindungen der Formel I können allein oder in Kombination mit pharmazeutisch anwendbaren Trägermaterialien in vielen Dosierungsformen eingesetzt werden. Zum Beispiel kann man feste Präparate wie Tabletten, Kapseln, Pulver, Granulate, Suppositorien, Dragees und dergleichen für die orale Verabreichung einsetzen. Feste Präparate können einen anorganischen Trägerstoff wie Talkum oder einen organischen Trägerstoff wie Milchzucker oder Stärke enthalten. Zusätze wie Magnesiumstearat (ein Gleitmittel) können ebenfalls eingesetzt werden. Flüssige Präparate wie Lösungen, Suspensionen oder Emulsionen können die üblichen Verdünnungsmittel wie Wasser, Vaseline, Suspensionsmittel wie Polyoxyäthylenglykol und dergleichen enthalten. Es können auch zusätzlich andere Bestandteile zugegeben werden wie Konservierungsmittel, Stabilisierungsmittel und Netzmittel.

Die nachfolgenden Beispiele erläutern die Herstellung der neuen Verbindungen der allgemeinen Formel I, sie sollen jedoch den Umfang der Erfindung in keiner Weise beschränken.

Die Strukturen der neuen Verbindungen wurden durch spektroskopische Untersuchungen, insbesondere durch eine genaue Analyse der NMR-Spektren gesichert. In den Tabellen werden, wenn keine weiteren Angaben vorliegen, die Schmelzpunkte der Monohydrochloride angegeben. Evtl. Vorliegen von eingeschlossenen Mengen an Wasser, Aceton, Äthanol oder dergleichen werden angeführt.

Wenn kein Salz angegeben ist, wurde für die öligen Basen im IR-Spektrum die Amid-C=O-Bande im Bereich 1630-1650 $cm^{-1}$ bestimmt (Gerät Perkin-Elmer IR-Spektrophotometer 157 G).

*Beispiel 1*

*1-Methyl-2-[(thiophen-2-carbonyl)-aminomethyl]-5-phenyl-1H-2,3-dihydro-1,4-benzodiazepin*

a) 202 g $N_1$-Benzoyl-$N_2$-methyl-$N_2$-phenyl-2-hydroxy-1,3-diaminopropan wurden in 1000 ml Phosphoroxidchlorid 2,5 Stunden unter Rückfluss gekocht. Dann wurde das überschüssige Phosphoroxidchlorid abdestilliert und der Rückstand in Chloroform (1000 ml) aufgenommen. Die Chloroformlösung wurde mit 1000 ml Eis/Wasser durchgerührt, die organische Phase wurde abgetrennt und 5- bis 6mal mit 200 ml Wasser gewaschen. Anschliessend wurde die organische Phase mit 1200 ml Natronlauge (20%) geschüttelt und darauf mit Wasser neutral gewaschen. Die Chloroformphase wurde dann durch Zusatz von Natriumsulfat und -Tonerde (Aluminiumoxid «Giulini») getrocknet und entfärbt. Nach dem Filtrieren wurde die Lösung eingedampft.

b) Die erhaltene Rohbase (202 g) des Gemisches aus 1-Methyl-2-chlormethyl-5-phenyl-1H-2,3-dihydro-1,4-benzodiazepin und 1-Methyl-3-chlor-6-phenyl-1,2,3-tetrahydro-benzodiazocin wurde in 1300 ml Methanol mit 138,5 g Kalium-Phthalimid und 38,2 g Kaliumjodid 22 Stunden unter Rückfluss erhitzt. Dann wurde das Methanol abdestilliert und der Rückstand mit 500 ml Chloroform versetzt. Die unlöslichen Anteile wurden abfiltriert und verworfen. Das Filtrat wurde eingedampft, wobei 256,4 g 1-Methyl-2-phthalimidomethyl-5-phenyl-1H-2,3-dihydro-1,4-benzodiazepin als öliger Rückstand erhalten wurden.

Dieser wurde ohne weitere Reinigung mit 71,1 g Hydrazinhydrat in 3200 ml Äthanol 4,5 Stunden unter Rückfluss gekocht. Dann wurden 330 ml konz. Salzsäure (32%) zugesetzt und die Reaktionsmischung weitere 15 Minuten unter Rückfluss erhitzt. Nach dem Abkühlen wurden die ausgeschiedenen Kristalle abfiltriert. Das Filtrat wurde anschliessend im Vakuum eingedampft. Der Rückstand wurde mit 1500 ml Wasser versetzt und noch einmal filtriert. Das Filtrat wurde mit Salzsäure versetzt und mit Methylenchlorid wurden alle nicht basischen Anteile entfernt. Durch Zugabe von konz. Natronlauge (50%) wurde die saure, wässrige Phase alkalisch gestellt. Die sich ölig ausscheidende Base wurde in Methylenchlorid aufgenommen, mit Kochsalzlösung (10%) mehrmals gewaschen, über Natriumsulfat getrocknet und filtriert.

Der nach Abdestillieren erhaltene Rückstand (127,3 g) wurde in Äther gelöst, filtriert und das Filtrat mit einer gesättigten Lösung von Chlorwasserstoff in Äther versetzt. Die ausgeschiedenen Kristalle wurden abfiltriert, mit Äther gewaschen und mit Aceton und wenig Isopropanol in der Kälte gerührt. Die Kristalle wurden abgesaugt, mit Aceton gewaschen und getrocknet.

Es wurden 108,5 g 1-Methyl-2-aminomethyl-5-phenyl-1H-2,3-dihydro-1,4-benzodiazepin-dihydrochlorid mit Fp 209-213°C erhalten.

c) 13,4 g 1-Methyl-2-aminomethyl-5-phenyl-1H-2,3-dihydro-1,4-benzodiazepin (erhalten, wie vorhergehend beschrieben) und 7,65 ml Triäthylamin wurden in 320 ml Methylenchlorid gelöst und unter Rühren und Kühlung mit Eis mit einer Lösung von 8,12 g Thiophen-2-carbonsäurechlorid in 20 ml Methylenchlorid versetzt. Anschliessend wurde die Reaktionslösung bei Raumtemperatur über Nacht stehen gelassen. Darauf wurde mit Wasser, Natriumcarbonatlösung (10%) und gesättigter Kochsalzlösung gewaschen, über Natriumsulfat getrocknet und filtriert. Nach dem Abdestillieren des Lösungsmittels wurden 21 g Substanz erhalten, die nacheinander mit Cyclohexan, Toluol, Methylenchlorid und Äthanol an 300 g Aluminiumoxid I (stand. Fa. Merck) chromatographiert wurden. Die Toluol- und Methylenchlorid-Eluate (19,4 g) wurden vereinigt, in Äther gelöst und mit einer Lösung von 7,8 g rac.-Weinsäure in Äthanol versetzt. Durch Zugabe von Äther wurde das Salz ausgefällt und nach der Filtration aus Äthanol bei −70 bis −60°C umkristallisiert.

Es wurden 10 g 1-Methyl-2-[(thiophen-2-carbonyl)-aminomethyl]-5-phenyl-1H-2,3-dihydro-1,4-benzodiazepintartrat × 0,4 Mol Äthanol mit Fp 110 bis 125°C (Zersetzung) erhalten. Die Base hatte einen Fp 112-115°C.

*Beispiel 2*

*1-Methyl-2-[(thiophen-3-carbonyl)-aminomethyl]-5-phenyl-1H-2,3-dihydro-1,4-benzodiazepin*

a) 68,4 g des nach Beispiel 1a erhaltenen Cyclisierungsgemisches aus 1-Methyl-2-chlormethyl-5-phenyl-1H-2,3-dihydro-1,4-benzodiazepin und 1-Methyl-3-chlor-6-phenyl-1,2,3,4-tetrahydro-benzodiazocin wurden mit 47,2 g Natriumazid in 450 ml Dimethylformamid 4 Stunden auf 100°C erwärmt. Danach wurde das Dimethylformamid im Vakuum abdestilliert und der Rückstand zwischen 300 ml Toluol und 200 ml Wasser verteilt. Die organische Phase wurde abgetrennt, mit Kochsalzlösung (10%) gewaschen, über Natriumsulfat getrocknet und filtriert. Dann wurde das Lösungsmittel abdestilliert (Rohprodukt 56,1 g).

Der Rückstand wurde in Äther gelöst und mit einer gesättigten Lösung von Chlorwasserstoff in Äther versetzt. Die ausfallenden Kristalle wurden abfiltriert und aus Aceton/Isopropanol umkristallisiert.

Es wurden 36,7 g 1-Methyl-2-azidomethyl-5-phenyl-1H-2,3-dihydro-1,4-benzodiazepinhydrochlorid mit Fp 181-183°C erhalten.

b) 21,7 g 1-Methyl-2-azidomethyl-5-phenyl-1H-2,3-dihydro-1,4-benzodiazepinhydrochlorid wurden in 325 ml Methanol gelöst und nach Zugabe von 9,5 ml Triäthylamin mit 13,5 ml Hydrazinhydrat versetzt. Dann wurde die Reaktionslösung bei Raumtemperatur portionsweise unter Rühren mit 10 g Raney-Nickel versetzt. Die Raney-Nickel-Zugabe

war nach 3 Stunden beendet. Die Reaktionsmischung wurde noch 1 Stunde gerührt, dann wurde das Raney-Nickel abfiltriert. Das Filtrat wurde im Vakuum eingedampft, in Methylenchlorid aufgenommen und mit Wasser und Kochsalzlösung (10%) gewaschen. Dann wurde die organische Phase über Natriumsulfat getrocknet, filtriert und eingedampft. Es wurden 17,1 g 1-Methyl-2-aminomethyl-5-phenyl-1H-2,3-dihydro-1,4-benzodiazepin erhalten, dessen Dihydrochlorid einen Fp 209-213°C hatte.

c) 13,5 g Thiophen-3-carbonsäure wurden in 300 ml Methylenchlorid gelöst und auf 0 bis 5°C abgekühlt. Darauf wurden 14,5 ml Trimethylamin zugegeben und dann innerhalb von 5 bis 10 Minuten 11,2 ml Chlorameisensäureäthylester zugetropft. Die Reaktionslösung wurde anschliessend noch 30 Minuten bei 0 bis 5°C gerührt und dann unter Kühlung so zu einer Lösung von 27,9 g 1-Methyl-2-aminomethyl-5-phenyl-1H-2,3-dihydro-1,4-benzodiazepin in 200 ml Methylenchlorid getropft, dass die Temperatur zwischen 0 und 5°C blieb. Anschliessend wurde die Reaktionslösung noch 4 Stunden bei Raumtemperatur gerührt, mit Wasser, verdünnter Ammoniaklösung (10%) und Kochsalzlösung gewaschen, über Natriumsulfat getrocknet und filtriert. Dann wurde das Lösungsmittel abdestilliert. Es wurden 37,8 g Rohprodukt erhalten.

Die Rohbase wurde in Äther gelöst und mit einer gesättigten Lösung von Chlorwasserstoff in Äther versetzt. Die ausgefallenen Kristalle wurden abfiltriert und mit Aceton/Essigsäureäthylester ausgekocht. Es wurden 21,9 g 1-Methyl-2-[(thiophen-3-carbonyl)-aminomethyl]-5-phenyl-1H-2,3-dihydro-1,4-benzodiazepinhydrochlorid mit Fp 234 bis 237,5°C erhalten.

*Beispiel 3*

*7-Nitro-1-methyl-2-[(thiophen-2-carbonyl)-aminomethyl]-5-phenyl-1H-2,3-dihydro-1,4-benzodiazepin*

2,5 g 1-Methyl-2-[(thiophen-2-carbonyl)-aminomethyl]-5-phenyl-1H-2,3-dihydro-1,4-benzodiazepin wurden in 9 ml Acetanhydrid gelöst und bei 30 bis 35°C portionsweise mit 1,58 g Kupfer(II)-nitrat . $3H_2O$ versetzt. Nach beendeter Zugabe des Kupfersalzes wurde die Reaktionsmischung auf eine gesättigte Natriumbicarbonatlösung und Eis gegeben. Die alkalische Lösung wurde mit Methylenchlorid (50 ml) extrahiert und in üblicher Weise aufgearbeitet.

Nach Überführung in das Hydrochlorid wurden 0,7 g 7-Nitro-1-methyl-2-[(thiophen-2-carbonyl)-aminomethyl]-5-phenyl-1H-2,3-dihydro-1,4-benzodiazepin-hydrochlorid mit Fp 254-256°C erhalten.

*Beispiel 4*

*7-Nitro-1-methyl-2-[(thiophen-2-carbonyl)-aminomethyl]-5-phenyl-1H-2,3-dihydro-1,4-benzodiazepin*

Eine Lösung von 5,2 g 1-Methyl-2-chlormethyl-5-phenyl-1H-2,3-dihydro-1,4-benzodiazepin in 50 ml Eisessig wurde bei 5°C mit einer Lösung von 4,5 g Kaliumnitrat in 8 ml konz. Schwefelsäure versetzt. Das Reaktionsgemisch wurde 1 Stunde bei Raumtemperatur gerührt, dann auf 200 g Eis gegossen, mit verdünnter Natronlauge (20%) alkalisch gestellt und mit Methylenchlorid extrahiert. Anschliessend wurde die organische Phase mit Wasser neutral gewaschen, über Natriumsulfat getrocknet und filtriert. Nach dem Abdestillieren des Lösungsmittels wurde 7-Nitro-1-methyl-2-chlormethyl-5-phenyl-1H-2,3-dihydro-1,4-benzodiazepin erhalten, dessen Hydrochlorid Fp 213-214°C hatte.

4,5 g dieser Verbindung wurden in 50 ml Methanol mit 2,7 g Kaliumphthalimid und 800 mg Kaliumjodid unter Rückfluss erhitzt. Nach üblichem Aufarbeiten wurden 4 g 7-Nitro-2-methal-2-phthalimidomethyl-5-phenyl-1H-2,3-dihydro-1,4-benzodiazepin erhalten. Diese wurden mit 300 ml Salzsäure (24%) 4 Stunden unter Rückfluss erhitzt. Die Reaktionslösung wurde eingeengt und vorsichtig mit Natriumhydroxidlösung bis zur alkalischen Reaktion versetzt. Dann wurde wie üblich mit Methylenchlorid das rohe Reaktionsprodukt (2 g 7-Nitro-1-methyl-2-aminomethyl-5-phenyl-1H-2,3-dihydro-1,4-benzodiazepin) isoliert und ohne Reinigung in 100 ml Methylenchlorid gelöst. Nach Zugabe von 0,65 g Triäthylamin wurde unter Eiskühlung mit einer Lösung von 0,93 g Thiophen-2-carbonsäurechlorid in 20 ml Methylenchlorid versetzt. Nach üblicher Aufarbeitung wurden 1,8 g 7-Nitro-1-methyl-2-[(thiophen-2-carbonyl)-aminomethyl]-1H-2,3-dihydro-1,4-benzodiazepinhydrochlorid mit Fp 254-256°C erhalten.

*Beispiel 5*

*7-Chlor-1-methyl-2-[(furan-2-carbonyl)-aminomethyl]-5-phenyl-1H-2,3-dihydro-1,4-benzodiazepin*

4,2 g 1-Methyl-2-chlormethyl-5-phenyl-1H-2,3-dihydro-1,4-benzodiazepin und 1,95 g N-Chlorsuccinimid wurden in 75 ml Methylenchlorid 24 Stunden unter Rückfluss gekocht. Dann wurde die Reaktionslösung mit Wasser gewaschen und über Natriumsulfat getrocknet. Nach dem Filtrieren und Abdestillieren des Lösungsmittels wurden 4,5 g 7-Chlor-1-methyl-2-chlormethyl-5-phenyl-1H-2,3-dihydro-1,4-benzodiazepin erhalten, dessen Hydrochlorid Fp 110-112°C (aus Isopropanol) hatte.

4,0 g dieserVerbindung wurden in25 ml Methanol mit 2,5 g Kaliumphthalimid und 750 mg Kaliumjodid 20 Stunden unter Rückfluss erhitzt. Dann wurde das Methanol abdestilliert und der Rückstand mit 25 ml Chloroform versetzt. Die unlöslichen Anteile wurden abfiltriert und verworfen. Das Filtrat wurde eingedampft und der Rückstand (6,3 g) mit 1,9 g Hydrazinhydrat in 100 ml Äthanol 4 Stunden unter Rückfluss erhitzt. Darauf wurden 10 ml konz. Salzsäure (32%ig) zugesetzt und die Reaktionsmischung weitere 15 Minuten unter Rückfluss erhitzt. Nach dem Abkühlen wurden die ausgeschiedenen Kristalle abfiltriert und das Filtrat im Vakuum eingedampft. Der Rückstand wurde mit 50 ml Wasser versetzt und nochmals filtriert. Das Filtrat wurde mit konz. Salzsäure angesäuert und mit Methylenchlorid (100 ml) extrahiert. Durch Zugabe von konz. Natriumhydroxidlösung (50%ig) wurde die Base ölig ausgefällt, in Methylenchlorid gelöst, mit gesättigter Natriumchloridlösung gewaschen, über Natriumsulfat getrocknet und filtriert. Nach dem Abdestillieren des Lösungsmittels wurden 3,1 g 7-Chlor-1-methyl-2-aminomethyl-5-phenyl-1H-2,3-dihydro-1,4-benzodiazepin erhalten.

Der Rückstand wurde ohne weitere Reinigung in 80 ml Methylenchlorid gelöst und mit 1,05 g Triäthylamin versetzt. Dann wurde unter Kühlen mit Eis unter Ausschluss von Feuchtigkeit eine Lösung von 1,35 g Furan-2-carbonsäurechlorid in 10 ml Methylenchlorid zugetropft. Anschliessend wurde die Reaktionslösung noch 2 Stunden bei Raumtemperatur gerührt und wie üblich aufgearbeitet.

Nach Überführung in das Hydrochlorid wurden 2,67 g 7-Chlor-1-methyl-2-[(furan-2-carbonyl)-aminomethyl]-5-phenyl-1H-2,3-dihydro-1,4-benzodiazepinhydrochlorid mit Fp 235-236,5°C erhalten.

*Beispiel 6*

*1-Methyl-2-[(furan-3-carbonyl)-aminomethyl]-5-[(2'-fluorphenyl)-1H-2,3-dihydro-1,4-benzodiazepin*

12,5 g 1-Methyl-2-aminomethyl-5-(2'-fluorphenyl)-1H-2,3-dihydro-1,4-benzodiazepin-dihydrochlorid wurden in 160 ml Methylenchlorid unter Zugabe von 15,9 ml Triäthylamin gelöst. Dann wurde unter Eiskühlung eine Lösung von 5,0 g Furan-3-carbonsäurechlorid in 50 ml Methylenchlorid zugetropft. Nachdem die Reaktionslösung noch 3 Stunden bei Raumtemperatur gerührt worden war, wurde wie üblich aufgearbeitet. Es wurden 9,0 g des Hydrochlorids mit Fp 227-228°C erhalten.

*Beispiel 7*

*8-Methoxy-1-methyl-2-[(1-methylpyrrol-2-carbonyl)-aminomethyl]-5-(2'-chlorphenyl)-1H-2,3-dihydro-1,4-benzodiazepin*

9,2 g 8-Methoxy-1-methyl-2-aminomethyl-5-(2'-chlorphenyl)-1H-2,3-dihydro-1,4-benzodiazepin wurden in 230 ml Methylenchlorid gelöst und mit 4,0 ml Triäthylamin versetzt. Dann wurde eine Lösung von 4,0 g 1-Methylpyrrol-2-carbonsäurechlorid in 25 ml Methylenchlorid unter Eiskühlung zugetropft. Nach beendeter Zugabe wurde sofort wie üblich aufgearbeitet. Es wurden 2,4 g des Hydrochlorids, das 0,66 Mol $H_2O$ enthielt, mit Fp 178-225°C (unter Zersetzung) erhalten.

*Beispiel 8*

*2-[(Thiophen-2-carbonyl)-aminomethyl]-5-phenyl-1H-2,3-dihydro-1,4-benzodiazepin*

10 g 1-Methyl-2-aminomethyl-5-phenyl-1H-2,3-dihydro-1,4-benzodiazepin wurden in 40 ml Jodwasserstoffsäure (67%) unter Rühren für 4 Stunden auf 80°C erwärmt. Anschliessend wurde die Reaktionsflüssigkeit auf Eis (500 g) gegossen und mit festem Natriumcarbonat vorsichtig neutralisiert. Nach Zusatz von 50 ml konz. Natronlauge wurde die Base mit Methylenchlorid extrahiert. Die organische Phase wurde mit gesättigter Kochsalzlösung gewaschen, über Natriumsulfat getrocknet und filtriert. Nach dem Abdestillieren des Lösungsmittels wurden 9 g ölige Base an 2-Aminomethyl-5-phenyl-1H-2,3-dihydro-1,4-benzodiazepin erhalten.

Die ölige Base wurde in 250 ml Methylenchlorid unter Zusatz von 3,6 g Triäthylamin gelöst und unter Rühren und Kühlen mit einer Lösung von 5,2 g Thiophen-2-carbonsäurechlorid in 25 ml Methylenchlorid tropfenweise versetzt. Die Reaktionslösung wurde 2 Stunden bei Raumtemperatur gerührt und aufgearbeitet. Es wurden 20 g öliger Rückstand erhalten, der mit Toluol an 200 g Aluminiumoxid II chromatographiert wurde. In 200-ml-Fraktionen wurde in den Fraktionen 10 bis 24 das gewünschte Reaktionsprodukt angereichert und nach dem Verdampfen des Toluols gemeinsam in 200 ml Methylenchlorid aufgenommen. Die Methylenchloridlösung wurde mt 50 g γ-Tonerde gerührt, filtriert und das Filtrat eingedampft. Als Rückstand wurden 14,2 g Rohprodukt erhalten, das aus Toluol umkristallisiert wurde. Es wurden 6,7 g der Base mit Fp 172-174°C erhalten.

*Beispiel 9*

*1-Methyl-2-[(thiophen-2-carbonyl)-N-methylaminomethyl]-5-phenyl-1H-2,3-dihydro-1,4-benzodiazepin*

7,6 g 1-Methyl-2-[(thiophen-2-carbonyl)-aminomethyl]-5-phenyl-1H-2,3-dihydro-1,4-benzodiazepin wurden in 100 ml Tetrahydrofuran gelöst und unter Rühren bei Raumtemperatur mit 0,66 g Natriumhydrid (80% in Öl) versetzt. Anschliessend wurde die Reaktionsmischung auf 5°C abgekühlt und langsam mit einer Lösung von 1,36 ml Methyljodid in 10 ml Tetrahydrofuran versetzt (Zutropfzeit ca. 30 Minuten). Die Reaktionslösung wurde anschliessend 2 Stunden bei 5 bis 10°C gerührt, danach mit 10 ml Eiswasser und soviel Toluol verdünnt, dass nach Zugabe von weiterem Wasser zwei Phasen gebildet wurden. Die organische Phase wurde mehrfach mit Wasser gewaschen, über Natriumsulfat getrocknet und filtriert. Nach dem Abdestillieren des Lösungsmittels wurden 7,5 g Rohprodukt erhalten.

Das Rohprodukt wurde in 50 ml trockenem Äther gelöst und mit einer gesättigten Lösung von 7,8 g D,L-Weinsäure in Äthanol versetzt. Durch weiteren Zusatz von Äther wurde das Tartrat ausgefällt, abfiltriert und aus Äthanol umkristallisiert.

Es wurden 9 g 1-Methyl-2-[(thiophen-2-carbonyl)-N-methylaminomethyl]-5-phenyl-1H-2,3-dihydro-1,4-benzodiazepintartrat × 0,4 Mol Äthanol mit Fp 110-125°C erhalten.

*Beispiel 10*

*1-Methyl-2-[(thiophen-2-carbonyl)-N-n-propylaminomethyl]-5-phenyl-1H-2,3-dihydro-1,4-benzodiazepin*

10 g des nach Beispiel 1a aus $N_1$-Benzoyl-$N_2$-methyl-$N_2$-phenyl-2-hydroxy-1,3-diaminopropan durch Cyclisierung mit Phosphoroxidchlorid erhaltenen Produkts wurden mit 100 ml n-Propylamin 24 Stunden im Autoklaven auf 80°C erhitzt. Nach dem Abkühlen wurde das überschüssige Amin abdestilliert. Der Rückstand wurde in Wasser aufgenommen und nach Zusatz von 50 ml verdünnter Natronlauge (20%) mit Methylenchlorid extrahiert. Die organische Phase wurde mit gesättigter Kochsalzlösung

gewaschen, über Natriumsulfat getrocknet und filtriert. Nach dem Abdestillieren des Lösungsmittels wurden 8 g 1-Methyl-2-propylaminomethyl-5-phenyl-1H-2,3-dihydro-1,4-benzodiazepin als öliger Rückstand erhalten.

Der Rückstand wurde in 150 ml Methylenchlorid unter Zusatz von 2,6 g Triäthylamin gelöst und unter Rühren und Kühlen mit Eis mit einer Lösung von 3,8 g Thiophen-2-carbonsäurechlorid in 20 ml Methylenchlorid versetzt. Die Reaktionslösung wurde 2 Stunden bei Raumtemperatur gerührt, darauf mit Wasser, Natriumcarbonatlösung (10%) und gesättigter Natriumchloridlösung gewaschen. Nach Trocknen über Natriumsulfat und Filtration wurde das Lösungsmittel abdestilliert. Der Rückstand (9 g) wurde in Äthanol mit 2 Molen rac.-Weinsäure versetzt und aus Äthanol/Äther kristallisiert.

Es wurden 7 g der Base erhalten, welche mit 1,6 Mol Weinsäure und 0,5 Mol $H_2O$ kristallisierte. Fp: 108-118°C (unter Zersetzung).

Entsprechend den Beispielen 1 bis 10 können folgende Verbindungen hergestellt werden:

1-Methyl-2-[(thiophen-2-carbonyl)-aminomethyl]-5-phenyl-1H-2,3-dihydro-1,4-benzodiazepin--Derivate der allgemeinen Formel I, in welchen die Substituenten in den Ringen A und B und $R_4$ folgende Bedeutung haben:

| Substituenten in A | B | $R_4$ | Schmelzpunkt °C Hydrochlorid |
|---|---|---|---|
| H | H | H | 112-115 |
| 8-Cl | H | H | 240-242 1,5 HCl |
| 7-F | H | H | 224-227 |
| 8-F | H | H | 232-236 |
| 7-$CH_3$ | H | H | 219-224 |
| 6,8-di-$CH_3$ | H | H | 232-234 |
| 7,8-di-$CH_3$ | H | H | 246-249 |
| 8-$C_2H_5$ | H | H | 221-223,5 |
| 7-i-$C_3H_7$ | H | H | 262-264 |
| 7-$OCH_3$ | H | H | 150-157 . 0,5 $H_2O$ |
| 8-$OCH_3$ | H | H | 230,5-231,5 |
| 7,8-di-$OCH_3$ | H | H | 243-245 |
| 6,8-di-$OCH_3$ | H | H | 245-246,5 |
| 8-$OC_2H_5$ | H | H | 195-197 . 1 Aceton |
| 7-n-$OC_3H_7$ | H | H | 227-230 1,4 HCl |
| 7,8-$OCH_2O$- | H | H | 259-263 |
| 7,8-$OC_2H_4O$- | H | H | 265-276 |
| 8-$SCH_3$ | H | H | 259-265 |
| H | 2-F | H | 241-242 |
| H | 3-$OCH_3$ | H | 205-208 |
| H | 2,6-di-$OCH_3$ | H | 202-205 |
| H | 3,4-di-$OCH_3$ | H | 218-220 |
| 7-$CH_3$ | 2-F | H | 238-249 |
| 8-$OCH_3$ | 3-F | H | 236-238 |
| 8-$OCH_3$ | 4-F | H | 226-229 |
| 8-$OCH_3$ | 2-F | H | 213-216 |
| 8-$OCH_3$ | 2-Cl | H | 220-229 |
| 8-$OCH_3$ | 2-Br | H | 229-232 |
| 8-$OCH_3$ | 2-$CF_3$ | H | 173-177 |
| 8-$OCH_3$ | 3-$CF_3$ | H | 224-227 |
| 8-$OCH_3$ | 4-$CF_3$ | H | 224-227 |
| 8-$OCH_3$ | 2-$CH_3$ | H | 229-232 |
| 7-F | 3,4,5-tri-$OCH_3$ | H | 201-203 |
| 7-$CH_3$ | 3,4,5-tri-$OCH_3$ | H | 178-182 |
| 7-Br | 2-Cl | H | 246-247,5 |
| 7-$CF_3$ | H | H | 254-256 |
| 7,8-$OCH_2O$- | 2-F | H | |
| H | H | 5-$C_2H_5$ | 192-195 |
| 8-$OCH_3$ | 2-F | 5-$C_3H_5$ | 171-174 |
| H | H | 4-$CH_3$ | 221-223 . 0,75 Aceton |

TABELLE (Fortsetzung)

| Substituenten in A | B | $R_4$ | Schmelzpunkt °C Hydrochlorid |
|---|---|---|---|
| H | H | 3-$CH_3$ | 227-228 |
| 8-$OCH_3$ | H | 3-$CH_3$ | 226-227 |
| 7-n-$OC_3H_7$ | H | 3-$CH_3$ | 186-189 |
| 8-$OCH_3$ | 2-F | 3-$CH_3$ | 161-165 . 0,5 $H_2O$ |
| 8-$OCH_3$ | 2-F | 5-$CH_3$ | 180-189 (Zers.) . 0,25 $H_2O$ |
| 8-$OCH_3$ | 2-F | 4-$CH_3$ | 198-202 . 0,15 $H_2O$ |
| H | H | 4-Br | 202-205 |
| H | H | 5-Br | 236-245 |
| 8-$OCH_3$ | H | 5-Br | 225-226 |
| 8-$OCH_3$ | 2-F | 5-Br | 219,5-220,5 |
| 8-$OCH_3$ | 3-F | 5-Br | 221-222 |
| 8-$OCH_3$ | 4-F | 5-Br | 228-231 |
| H | H | 5-$CH_3$ | 218-223 . 0,4 Aceton |
| 8-F | H | 5-$CH_3$ | 238-240 |
| 7-$OCH_3$ | H | 5-$CH_3$ | 246-251 |
| 8-$OCH_3$ | H | 5-$CH_3$ | 231-233 |
| 8-$OC_2H_5$ | H | 5-$CH_3$ | 238-240 |
| 7,8-$OCH_2O$- | H | 5-$CH_3$ | 257-267 |
| 8-$SCH_3$ | H | 5-$CH_3$ | 229-232 |
| 7-$CH_3$ | 2-F | 5-$CH_3$ | 238-247 |
| 7-F | 3,4,5-$OCH_3$ | 5-$CH_3$ | 170-172 |
| H | H | 5-$OCH_3$ | Öl |
| H | H | 5-$OC_2H_5$ | Öl |
| H | H | 5-Cl | Öl |
| 8-$OCH_3$ | 4-F | 5-$CH_3$ | Öl |
| 8-$OCH_3$ | 4-F | 4-$CH_3$ | Öl |
| 7,8-$OCH_2O$- | 2-F | 5-$CH_3$ | Öl |
| 7,8-$OCH_2O$- | 2-F | 4-$CH_3$ | Öl |
| 7,8-$OCH_2O$- | 2-F | 5-$OCH_3$ | Öl |
| H | 2-F | 5-$OCH_3$ | Öl |
| H | 2-F | 4-Br | 204-208 |

1-Methyl-2-[(furan-3-carbonyl)-aminomethyl]-5--phenyl-1H-2,3-dihydro-1,4-benzodiazepin-Derivate der allgemeinen Formel I, in welchen die Substituenten in den Ringen A und B und $R_4$ folgende Bedeutung haben:

| Substitution in A | B | $R_4$ | Schmelzpunkt °C Hydrochlorid |
|---|---|---|---|
| 7-$CH_3$ | H | H | 244-245,5 2 HCl |
| 6,8-di-$CH_3$ | H | H | 227-229 |
| 7,8-di-$CH_3$ | H | H | 245-247 |
| 8-$C_2H_5$ | H | H | 209-212 |
| 7-i-$C_3H_7$ | H | H | 247-252 |
| 7-$OCH_3$ | H | H | 127-130 1,34 HCl . 0,45 $H_2O$ |
| 8-$OCH_3$ | H | H | 222-224 |
| 7,8-di-$OCH_3$ | H | H | 227-229 |
| 8-$OC_2H_5$ | H | H | 160-166 . 0,9 Aceton |
| 7-n-$C_3H_7$ | H | H | 227-230 |
| 7,8-$OCH_2O$- | H | H | 264-269 |
| 8-$SCH_3$ | H | H | 243-246 |
| H | 2-F | H | 242-243 |

TABELLE (Fortsetzung)

| Substituenten in A | B | $R_4$ | Schmelzpunkt °C Hydrochlorid |
|---|---|---|---|
| H | 3-$OCH_3$ | H | 205-207,5 |
| H | 3,4-di-$OCH_3$ | H | 216-217 |
| 7-Br | 2-Cl | H | 259-263 |
| 7-F | 3,4,5-tri-$OCH_3$ | H | 219-223 |
| 7-$CH_3$ | 3,4,5-tri-$OCH_3$ | H | 184-187 |
| H | 3-$CF_3$ | H | 231-232 |
| 8-$OCH_3$ | 4-$OCH_3$ | H | 218-222 |
| 7-Cl,8-$OCH_3$ | 2-F | H | Öl |
| 8-$CH_3$ | 2-F | H | Öl |
| 8-$NO_2$ | H | H | Öl |
| 8-$OC_2H_5$ | 2-F | H | Öl |
| 8-i-$OC_3H_7$ | 2-F | H | Öl |
| 7,8-$OCH_2O$- | 2-F | H | Öl |
| 6-$OCH_3$ | 2-F | H | Öl |
| 7-F,8-$OCH_3$ | 2-F | H | Öl |
| 6,7-$OCH_2O$- | 2-F | H | Öl |
| H | 2-F | H | Öl |
| H | 4-$CF_3$ | H | Öl |
| 6-OH | 2-F | H | Öl |
| 9-$CH_3$ | H | H | Öl |
| 8-$CH_3$ | H | H | Öl |
| H | H | 2-$CH_3$ | Öl |
| H | 2-F | 2-$CH_3$ | Öl |
| 8-$CH_3$,6-$OCH_3$ | 2-F | H | Öl |

1-Methyl-2-[(thiophen-3-carbonyl)-aminomethyl]-5-phenyl-1H-2,3-dihydro-1,4-benzodiazepin-Derivate der allgemeinen Formel I, in welchen die Substituenten in den Ringen A und B und $R_4$ folgende Bedeutung haben:

| Substitution in A | B | $R_4$ | Schmelzpunkt °C Hydrochlorid |
|---|---|---|---|
| H | H | H | 22-225 |
| 7-$CH_3$ | H | H | 238-240 |
| 8-$C_2H_5$ | H | H | 217,5-219 |
| 7-i-$C_3H_7$ | H | H | 245-251 |
| 7-$OCH_3$ | H | H | 219-222<br>0,25 $H_2O$ |
| 6,8-di-$OCH_3$ | H | H | 242-244 |
| 7,8-di-$OCH_3$ | H | H | 242,5-244 |
| 7,8-$OCH_2O$- | H | H | 258-265 |
| 8-$SCH_3$ | H | H | 246-249 |
| H | 2-F | H | 225-227 |
| H | 3-$OCH_3$ | H | 195-197 |
| H | 3,4-di-$OCH_3$ | H | 215-217<br>0,3 $H_2O$ |
| 7-Br | 2-Cl | H | 237-239 |
| 7-F | 3,4,5-tri-$OCH_3$ | H | 168-172 |
| 7-$CH_3$ | 3,4,5-tri-$OCH_3$ | H | 191-197 |
| 7,8-di-$CH_3$ | H | H | 241-244 |
| 6,8-di-$CH_3$ | H | H | 232,5-234 |
| H | H | 5-$CH_3$ | 196-198 |
| 8-$OCH_3$ | H | 5-$CH_3$ | 228-230 |
| 7-n-$OC_3H_7$ | H | 5-$CH_3$ | 216-219 |
| H | H | 5-$NO_2$ | 199-200<br>(Base) |

1-Methyl-2-[(furan-2-carbonyl)-aminomethyl]-5-phenyl-1H-2,3-dihydro-1,4-benzodiazepin-Derivate der allgemeinen Formel I, in welchen die Substituenten in den Ringen A und B und $R_4$ folgende Bedeutung haben:

| Substitution in A | B | $R_4$ | Schmelzpunkt °C Hydrochlorid |
|---|---|---|---|
| H | H | H | 135-137 (Base) |
| 7-Cl | H | H | 251-253 1,1 HCl |
| 8-Cl | H | H | 229-231 |
| 7-F | H | H | 224-226 |
| 8-F | H | H | 235-236 |
| 7-$CH_3$ | H | H | 224-226 |
| 7,8-di-$CH_3$ | H | H | 243,5-247 |
| 6,8-di-$CH_3$ | H | H | 231-232 |
| 8-$C_2H_5$ | H | H | 201-204 |
| 7-i-$C_3H_7$ | H | H | 243-246 . 0,2 $H_2O$ |
| 7-$OCH_3$ | H | H | 132-138 1,5 HCl . 0,3 $H_2O$ |
| 8-$OCH_3$ | H | H | 213-216 |
| 8-$OC_2H_5$ | H | H | 147-152 . 0,5 $H_2O$ . 0,5 Aceton |
| 7-n-$OC_3H_7$ | H | H | 214-218 |
| 7,8-di-$OCH_3$ | H | H | 240-241,5 |
| 7,8-$OCH_2O$- | H | H | 201-204 (Base) |
| 7,8-$OCH_2CH_2O$- | H | H | 267-275 |
| 8-$SCH_3$ | H | H | 247-249 |
| H | 2-F | H | 227-228 |
| H | 3-$CF_3$,4-Cl | H | 184-186 . 0,6 $H_2O$ . 0,3 Aceton |
| H | 3-$OCH_3$ | H | 183-186 |
| H | 2,6-di-$OCH_3$ | H | 198-202 |
| H | 3,4-di-$OCH_3$ | H | 215-217 |
| 7-$CH_3$ | 2-F | H | 236-243 1,4 HCl . 0,5 $H_2O$ . 0,2 Essigester |
| 8-$OCH_3$ | 2-Cl | H | 220-222 . 0,5 $H_2O$ |
| 8-$OCH_3$ | 2-Br | H | 228-233 . 0,5 $H_2O$ |
| 8-$OCH_3$ | 3-F | H | 234-236 |
| 8-$OCH_3$ | 4-F | H | 229-232 |
| 8-$OCH_3$ | 2-F | H | 220-221 |
| 8-$OCH_3$ | 2-$CF_3$ | H | 219-222 . 1,3 $H_2O$ |
| 8-$OCH_3$ | 3-$CF_3$ | H | 179-181 (Base) |
| 8-$OCH_3$ | 4-$CF_3$ | H | 231-232 . 0,7 $H_2O$ |
| 8-$OCH_3$ | 2-$CH_3$ | H | 211-214 |
| 8-$OCH_3$ | 2,4-di-Cl | H | 229-233 |
| 7-F | 3,4,5-tri-$OCH_3$ | H | 218-221 |
| 7-$CH_3$ | 3,4,5-tri-$OCH_3$ | H | 164-167 |
| H | 2-$CF_3$ | H | 199-201 |
| H | 2,6-di-F | H | 228-229 |
| H | 2-Br | H | 218-220 |
| H | 3-$CF_3$ | H | 215-217 bzw. 194-195 |
| 8-$OCH_3$ | 2-$OCH_3$ | H | 206-209 |

TABELLE (Fortsetzung)

| Substitution in A | B | $R_4$ | Schmelzpunkt °C Hydrochlorid |
|---|---|---|---|
| 8-$OCH_3$ | 3-$OCH_3$ | H | 216-219 |
| 8-$OCH_3$ | 4-$OCH_3$ | H | 230-233 |
| H | 4-$CF_3$ | H | Öl |
| 8-$CH_3$ | 2-F | H | Öl |
| 8-$OCH_3$ | 2,6-di-F | H | Öl |
| 8-$NO_2$ | H | H | Öl |
| 8-$OC_2H_5$ | 2-F | H | Öl |
| 8-i-$OC_3H_7$ | 2-F | H | Öl |
| 7,8-$OCH_2O$- | 2-F | H | Öl |
| 6-$OCH_3$ | 2-F | H | Öl |
| 7-F,8-$OCH_3$ | H | H | Öl |
| 8-$OCH_3$ | 4-F | 2-$CH_3$ | Öl |
| 8-$OCH_3$ | 4-F | 5-$CH_3$ | Öl |
| H | H | 2-$CH_3$ | Öl |
| H | H | 5-$CH_3$ | Öl |
| 8-i-$OC_3H_7$ | 2-F | 2-$CH_3$ | Öl |
| 8-i-$OC_3H_7$ | 2-F | 5-$CH_3$ | Öl |
| 7,8-$OCH_2O$- | 2-F | 2-$CH_3$ | Öl |
| 7,8-$OCH_3O$- | 2-F | 5-$CH_3$ | Öl |
| H | 2-F | 2-$CH_3$ | Öl |
| H | 2-F | 5-$CH_3$ | Öl |
| 8-$CH_3$ | H | H | Öl |
| 8-$CH_3$, 6-$OCH_3$ | 2-F | H | Öl |
| 7-$CH_3$,9-Cl | H | H | Öl |

2-(Acylaminomethyl)-5-phenyl-1H-2,3-dihydro--1,4-benzodiazepin-Derivate der allgemeinen Formel I, in welchen die Substituenten in den Ringen A und B und $R_1$, $R_2$, $R_3$ und $R_4$ folgende Bedeutung haben:

| $R_3$ | $R_1$ | $R_2$ | $R_4$ | A | B | | Schmelzpunkt °C |
|---|---|---|---|---|---|---|---|
| Thiophen-2- | $C_2H_5$ | H | H | H | H | HCl | 238-241 |
| » | $CH_3$ | $C_2H_5$ | H | H | H | Base | 115-117 |
| » | $CH_3$ | $C_2H_5$ | H | 8-$OCH_3$ | H | 1,2 HCl . 0,5 Aceton | 102-115 |
| » | $CH_3$ | n-$C_3H_7$ | H | H | H | 1,6 Tartrat, 0,5 $H_2O$ | 108-118 |
| » | $CH_3$ | Allyl | H | H | H | Base | 112-114 |
| » | $CH_3$ | $CH_3$ | H | H | H | 1,4 Tartrat | 96-105 |
| Thiophen-3- | $C_2H_5$ | H | H | 8-$OCH_3$ | 2,4-di-F | Base | Öl |
| | $C_2H_5$ | H | 2-$CH_3$ | 8-$OCH_3$ | 2-F | HCl | 199-201 |
| | $C_2H_5$ | H | 2-$CH_3$ | H | 2-F | Base | Öl |
| » | $C_2H_5$ | H | H | H | H | HCl | 224,5-226,5 |
| » | $C_2H_5$ | H | H | 8-$OCH_3$ | 2-F | HCl | 223-225 |
| » | Cyclopropyl-methyl | H | H | 8-$OCH_3$ | H | Base | Öl |
| » | H | H | H | H | 2-F | Base | Öl |
| » | $C_2H_5$ | H | H | 8-$OCH_3$ | 2,3-di-Cl | Base | Öl |
| Furan-3 | $C_2H_5$ | H | H | H | H | HCl . 0,15 $H_2O$ | 246-247 |
| » | $C_2H_5$ | H | H | 8-$OCH_3$ | 2-F | HCl | 228-231 |
| » | n-$C_4H_9$ | H | H | 8-$OCH_3$ | 2-F | HCl | 204-206 |
| » | $CH_3$ | $CH_3$ | H | 8-$OCH_3$ | H | HCl | 186-189 |
| | $CH_3$ | $C_2H_5$ | H | 8-$OCH_3$ | H | 1,5 Tartrat | 95-110 |
| » | $CH_3$ | Allyl | H | 8-$OCH_3$ | H | Base | Öl |
| » | H | H | H | H | 2-F | Base | Öl |

1-Methyl-2-[($R_3$-2-carbonyl)-aminomethyl]-5-phenyl-1H-2,3-dihydro-1,4-benzodiazepin-Derivate der allgemeinen Formel I, in welchen die Substituenten in den Ringen A und B und $R_3$ folgende Bedeutung haben:

| A | B | $R_3$ | Schmelzpunkt °C Hydrochlorid |
|---|---|---|---|
| H | H | 1H-Pyrrol | 224-229 |
| 8-$OCH_3$ | H | » | 234-235 |
| H | 2-F | » | 250-252 |
| 8-$CH_3$ | 2-F | » | Öl |
| 7,8-$OCH_2O$- | 2-F | » | Öl |
| 8-$OCH_3$ | 4-$OCH_3$ | » | 234-235 |
| H | H | 1-Methylpyrrol | 232-238 |
| 8-$OCH_3$ | H | » | 233-235 |
| 8-$OCH_3$ | 2-Br | » | 176-191 (Zers.) . 0,4 $H_2O$ . 0,4 $C_2H_5OH$ |
| 8-$OCH_3$ | 2-$CH_3$ | » | 210-213 |
| 8-$OCH_3$ | 4-$OCH_3$ | » | 222-224 |
| 7,8-$OCH_2O$- | 2-F | » | Öl |
| H | 2-F | » | 174-184 |
| 8-$CH_3$ | 2-F | » | Öl |
| 8-$CH_3$ | H | » | Öl |
| H | H | 1,2-Dimethylpyrrol | Öl |

1-Methyl-2-(acylaminomethyl)-5-phenyl-1H-2,3-dihydro-1,4-benzodiazepin-Derivate der allgemeinen Formel I, in welchen die Substituenten in den Ringen A und B und die Acylgruppe $R_3CO$ folgende Bedeutung haben:

| A | B | $R_3CO$ | Schmelzpunkt °C Dihydrochlorid |
|---|---|---|---|
| 8-$OCH_3$ | H | Picolinoyl | 216-218 1,8 HCl |
| 7-Br | 2-Cl | » | 217,5-219 1 HCl |
| H | H | Nicotinoyl | 211-214 . 0,13 $H_2O$ |
| 8-$OCH_3$ | H | » | 164-175 . 0,4 $H_2O$ . 0,83 Aceton |
| 7-Br | 2-F | » | 245-248 |
| 7-Br | 2-Cl | » | 235-238 . 0,25 $H_2O$ |
| 7-Cl | 2-Cl | » | 231-233 |
| 8-$OCH_3$ | H | Isonicotinoyl | 231-234 . 0,4 $H_2O$ |
| 7-Br | 2-Cl | » | 243-244 |

*Beispiel 11*

Man stellt Tabletten in folgender Zusammensetzung her:

| | pro Tablette |
|---|---|
| 1-Methyl-2-[(thiophen-2-carbonyl)-aminomethyl]-5-phenyl-1H-2,3-dihydro-1,4-benzodiazepin | 25 mg |
| Maisstärke | 60 mg |
| Milchzucker | 130 mg |
| Gelatine (10% Lösung) | 6 mg |

Der Wirkstoff, die Maisstärke und der Milchzucker werden mit einer 10%igen Gelatine-Lösung eingedickt. Die Paste wird zerkleinert; das Granulat wird auf ein geeignetes Blech gebracht und bei 45°C getrocknet.

Das getrocknete Granulat wird durch eine Zerkleinerungsmaschine geleitet, in einem Mixer mit folgenden Ingredienzien vermischt:

| | |
|---|---|
| Talkum | 5 mg |
| Magnesiumstearat | 5 mg |
| Maisstärke | 9 mg |

und sodann zu Tabletten von 240 mg verpresst.

*Beispiel 12*

Man stellt Suppositorien mit den folgenden Bestandteilen her:

1-Methyl-2-[(thiophen-2-carbonyl)-aminomethyl]-5-phenyl-1H-2,3-dihydro-1,4-benzodiazepin 25 mg
Kakaobutter 1975 mg

Der Wirkstoff und die fein geriebene Suppositoriengrundmasse werden gründlich gemischt und dann geschmolzen. Aus der durch Rühren homogen gehaltenen Schmelze werden Suppositorien von 2 Gramm gegossen.

*Beispiel 13*

Eine parenterale Gebrauchsform wird mit den folgenden Bestandteilen hergestellt:

| | |
|---|---|
| 1-Methyl-2-[(thiophen-2-carbonyl)-aminomethyl]-5-phenyl-1H-2,3-dihydro-1,4-benzodiazepin | 10 % |
| Dimethylacetamid | 10 % |
| Propylenglykol | 50 % |
| Benzylalkohol | 1,5 % |
| Äthanol | 10 % |
| Wasser für Injektionszwecke | ad 1 ml |

Der Wirkstoff wird in Dimethylacetamid gelöst und mit Benzylalkohol, Propylenglykol, Äthanol und Wasser versetzt. Man filtriert durch ein Kerzenfilter, füllt in geeignete Ampullen, verschliesst und sterilisiert.

**Patentansprüche** für die Vertragsstaaten: BE, CH, DE, FR, GB, IT, LI, LU, NL, SE

1. 2-Acylaminomethyl-1H-2,3-dihydro-1,4-benzodiazepine der allgemeinen Formel I

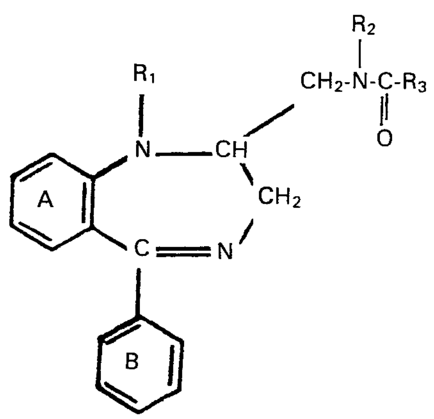

worin $R_1$ ist ein Wasserstoffatom oder eine Alkyl- oder Alkenylgruppe mit bis zu 4 Kohlenstoffatomen oder die Cyclopropylmethylgruppe,
$R_2$ ist ein Wasserstoffatom oder eine Alkyl- oder Alkenylgruppe mit bis zu 4 Kohlenstoffatomen,
$R_3$ ist eine Gruppe der Formel

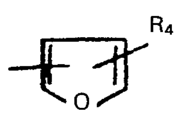 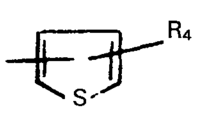 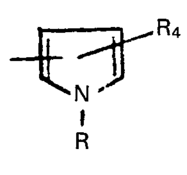

oder 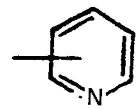

wobei R ist ein Wasserstoffatom oder ein $C_1$-$C_3$-Alkyl,
$R_4$ ist ein Wasserstoffatom, ein Halogenatom, insbesondere Chlor- oder Bromatom, eine Alkyl- oder Alkoxygruppe mit 1-4 Kohlenstoffatomen oder Nitro und
$R_4'$ ist Wasserstoff oder $C_1$-$C_4$-Alkyl,
A und B sind unabhängig voneinander unsubstituiert oder substituiert mit 1 bis 3 Substituenten, ausgewählt aus der Gruppe, bestehend aus Halogen, Alkylthio-, Alkoxy- oder Alkylgruppen mit 1-4 Kohlenstoffatomen, Hydroxy, Nitro und Trifluormethyl, oder
A und B sind Phenylreste, in welchen zwei benachbarte C-Atome durch eine Methylendioxy- oder Äthylendioxygruppe verbunden sind,
sowie deren optische Isomeren und die Säureadditionssalze der Verbindungen der allgemeinen Formel I.

2. 1-Methyl-2-[(thiophen-2-carbonyl)-aminomethyl]-5-phenyl-1H-2,3-dihydro-1,4-benzodiazepine der allgemeinen Formel I gemäss Anspruch 1, worin $R_1$ Methyl bedeutet, $R_2$ Wasserstoff bedeutet, $R_3$ eine 2-Thienylgruppe bedeutet, Ring B unsubstituiert ist und Ring A unsubstituiert ist oder substituiert durch 7-Fluor, 7-Methyl, 7-Isoprpyl, 6,8-Dimethyl, 7,8-Dimethyl, 7-Methoxy, 7-n-Propoxy, 7,8-Methylendioxy, 7,8-Äthylendioxy, 7-Trifluormethyl, 7-Nitro, 7,8-Dimethoxy, 6,8-Dimethoxy, 8-Fluor, 8-Chlor, 8-Äthyl, 8-Methylthio, 8-Methoxy oder 8-Äthoxy ist.

3. 1-Methyl-2-[(thiophen-2-carbonyl)-aminomethyl]-5-phenyl-1H-2,3-dihydro-1,4-benzodiazepine der allgemeinen Formel I gemäss Anspruch 1, worin $R_1$ Methyl, $R_2$ Wasserstoff und $R_3$ eine 2-Thienylgruppe bedeuten, Ring A unsubstituiert ist und Ring B unsubstituiert ist oder substituiert ist durch 2-Fluor, 3-Methoxy, 2,6-Dimethoxy oder 3,4-Dimethoxy.

4. 1-Methyl-2-[(thiophen-2-carbonyl)-aminomethyl]-5-phenyl-1H-2,3-dihydro-1,4-benzodiazepine der allgemeinen Formel I gemäss Anspruch 1, worin $R_1$ Methyl, $R_2$ Wasserstoff und $R_3$ eine 2-Thienylgruppe bedeuten, Ring A substituiert ist durch 7-Fluor, 7-Brom, 7-Methyl-, 8-Methoxy oder 7,8-Methylendioxy und Ring B substituiert ist durch 2-Fluor,3-Fluor, 4-Fluor, 2-Chlor, 2-Brom, 2-Methyl, 2-Trifluormethyl, 3-Trifluormethyl, 4-Trifluormethyl oder 3,4,5-Trimethoxy.

5. 1-Methyl-2-[(thiophen-2-carbonyl)-aminomethyl]-5-phenyl-1H-2,3-dihydro-1,4-benzodiazepine der allgemeinen Formel I gemäss Anspruch 1, worin $R_1$ Methyl und $R_2$ Wasserstoff bedeuten, Ring A unsubstituiert ist oder substituiert ist durch 7-Fluor, 8-Fluor, 7-Methyl, 8-Methylthio, 7-Methoxy, 8-Methoxy, 8-Äthoxy, 7-n-Propoxy oder 7,8-Methylendioxy und Ring B unsubstituiert ist oder substituiert ist durch 2-Fluor, 3-Fluor, 4-Fluor oder 3,4,5-Tri-

methoxy und $R_3$ eine durch $R_4$ substituierte 2-Thienylgruppe darstellt, worin $R_4$ 5-Chlor, 4-Brom, 5-Brom, 3-Methyl, 4-Methyl, 5-Methyl, 5-Äthyl, 5-Methoxy oder 5-Äthoxy bedeutet.

6. 1-Methyl-2-[(thiophen-3-carbonyl)-aminomethyl]-5-phenyl-1H-2,3-dihydro-1,4-benzodiazepine der allgemeinen Formel I gemäss Anspruch 1, worin $R_1$ Methyl, $R_2$ Wasserstoff und $R_3$ eine 3-Thienylgruppe bedeuten, Ring B unsubstituiert ist und Ring A unsubstituiert ist oder substituiert ist durch 7-Methyl, 8-Methyl, 9-Methyl, 8-Äthyl, 7-n-Propyl, 7-Isopropyl, 7-Methoxy, 8-Methoxy, 8-Äthoxy, 8-Methylthio, 6,8-Dimethyl oder 7,8-Dimethyl.

7. 1-Methyl-2-[(thiophen-3-carbonyl)-aminomethyl]-5-phenyl-1H-2,3-dihydro-1,4-benzodiazepin der allgemeinen Formel I gemäss Anspruch 1, worin $R_1$ Methyl und $R_2$ Wasserstoff bedeuten, Ring A unsubstituiert ist oder substituiert ist durch 7-Fluor, 7-Brom, 7-Chlor-8-methoxy, 7-Fluor-8-methoxy, 7-Methyl, 8-Methyl, 9-Methyl, 6-Methoxy, 8-Methoxy, 8-Äthoxy, 8-Isopropoxy, 8-Nitro, 8-Methyl-6-methoxy, 7,8-Methylendioxy, 6,7-Methylendioxy, 6-Hydroxy oder 7,8-Dimethoxy und Ring B unsubstituiert ist oder substituiert ist durch 2-Fluor, 2-Chlor, 3-Methoxy, 4-Methoxy, 3,4-Dimethoxy, 3,4,5-Trimethoxy, 3-Trifluormethyl oder 4-Trifluormethyl und $R_3$ eine durch $R_4$ substituierte 3-Thienylgruppe darstellt, worin $R_4$ Wasserstoff oder 2-Methyl bedeutet.

8. 1-Methyl-2-[(furan-2-carbonyl)-aminomethyl]-5-phenyl-1H-2,3-dihydro-1,4-benzodiazepine der allgemeinen Formel I gemäss Anspruch 1, worin $R_1$ Methyl und $R_2$ Wasserstoff bedeuten, Ring A unsubstituiert ist oder substituiert ist durch 7-Fluor, 7-Brom, 7-Methyl, 8-Äthyl, 7-Isopropyl, 8-Methylthio, 6,8-Dimethyl, 7,8-Dimethyl, 7-Methoxy, 8-Methoxy, 6,8-Dimethoxy, 7,8-Dimethoxy, 7-n-Propoxy, 7,8-Methylendioxy, 7-Chlor und Ring B unsubstituiert ist oder substituiert ist durch 2-Chlor, 2-Fluor, 3-Methoxy, 3,4-Dimethoxy, 3,4,5-Trimethoxy und $R_3$ eine durch $R_4$ substituierte 2-Furylgruppe darstellt, worin $R_4$ Wasserstoff, 5-Methyl oder 5-Nitro bedeutet.

9. 1-Methyl-2-[(furan-3-carbonyl)-aminomethyl]-5-phenyl-1H-2,3-dihydro-1,4-benzodiazepine der allgemeinen Formel I gemäss Anspruch 1, worin $R_1$ Methyl und $R_2$ Wasserstoff bedeuten, Ring A unsubstituiert ist oder substituiert ist durch 7-Chlor, 8-Chlor, 7-Fluor, 8-Fluor, 7-Fluor-8-methoxy, 8-Nitro, 7-Methyl-9-chlor, 8-Methylthio, 7-Methyl, 8-Methyl, 8-Methyl-6-methoxy, 8-Äthyl, 7-Isopropyl, 7,8-Dimethyl, 6,8-Dimethyl, 7-Methoxy, 8-Methoxy, 8-Äthoxy, 7-n-Propoxy, 8-Isopropoxy, 7,8-Dimethoxy, 7,8-Methylendioxy, 7,8-Äthylendioxy und Ring B unsubstituiert ist oder substituiert ist durch 2-Fluor, 3-Fluor, 4-Fluor, 2-Brom, 2-Chlor, 2-Methyl, 2-Trifluormethyl, 3-Trifluormethyl, 4-Trifluormethyl, 2,6-Difluor, 3-Trifluormethyl-4-chlor, 2,4-Dichlor, 2-Methoxy, 3-Methoxy, 4-Methoxy, 2,6-Dimethoxy, 3,4-Dimethoxy oder 3,4,5-Trimethoxy und $R_3$ eine durch $R_4$ substituierte 3-Furylgruppe darstellt, worin $R_4$ Wasserstoff, 2-Methyl oder 5-Methyl bedeutet.

10. 1-Methyl-2-[(pyrrol-2-carbonyl)-aminomethyl]-5-phenyl-1H-2,3-dihydro-1,4-benzodiazepine der allgemeinen Formel I gemäss Anspruch 1, worin $R_1$ Methyl und $R_2$ Wasserstoff bedeuten und Ring A unsubstituiert ist oder substituiert ist durch 8-Methyl, 8-Methoxy oder 7,8-Methylendioxy und Ring B unsubstituiert ist oder substituiert ist durch 2-Fluor, 2-Brom, 2-Methyl oder 4-Methoxy und $R_3$ einen 1H-Pyrrol-, 1-Methylpyrrol- oder 1,2-Dimethylpyrrol-Ring darstellt.

11. 1-Methyl-2-(acylaminomethyl)-5-phenyl-1H-2,3-dihydro-1,4-benzodiazepine der allgemeinen Formel I gemäss Anspruch 1, worin $R_1$ Methyl und $R_2$ Wasserstoff bedeuten, Ring A unsubstituiert ist oder substituiert ist durch 7-Brom, 7-Chlor oder 8-Methoxy und Ring B unsubstituiert ist oder substituiert ist durch 2-Fluor oder 2-Chlor und die Acylgruppe $R_3CO$ Picolinoyl, Nicotinoyl oder Isonicotinoyl darstellt.

12. 2-Acylaminomethyl-1H-2,3-dihydro-1,4-benzodiazepine entsprechend Anspruch 1, in welchen $R_1$ Wasserstoff, Methyl, Äthyl, n-Propyl, Isopropyl, n-Butyl oder Cyclopropylmethyl ist.

13. 2-Acylaminomethyl-1H-2,3-dihydro-1,4-benzodiazepine entsprechend Anspruch 1, in welchen $R_2$ Wasserstoff, Methyl, Äthyl, n-Propyl oder Allyl ist.

14. Verfahren zur Herstellung von 2-Acylaminomethyl-1H-2,3-dihydro-1,4-benzodiazepinen der allgemeinen Formel I gemäss Anspruch 1

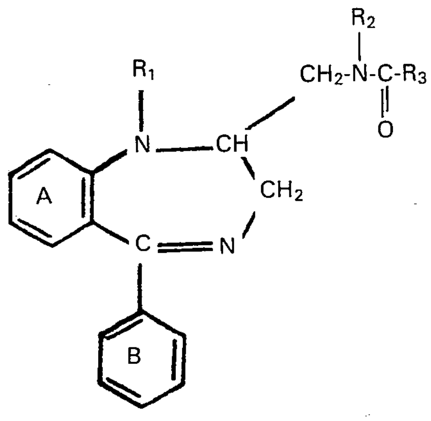

worin $R_1$ ist ein Wasserstoffatom oder eine Alkyl- oder Alkenylgruppe mit bis zu 4 Kohlenstoffatomen oder die Cyclopropylmethylgruppe,
$R_2$ ist ein Wasserstoffatom oder eine niedermolekulare Alkyl- oder Alkenylgruppe mit bis zu 4 Kohlenstoffatomen,
$R_3$ ist eine Gruppe der Formel

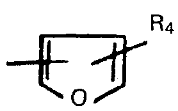 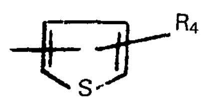 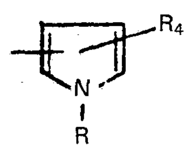

oder 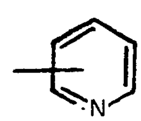

wobei R ist ein Wasserstoffatom oder ein $C_1$-$C_3$-Alkyl,

$R_4$ ist ein Wasserstoffatom, ein Halogenatom, insbesondere Chlor- oder Bromatom, eine Alkyl- oder Alkoxygruppe mit 1-4 Kohlenstoffatomen oder Nitro und
$R_4'$ ist Wasserstoff oder $C_1$-$C_4$-Alkyl,
A und B sind unabhängig voneinander unsubstituiert oder substituiert mit 1 bis 3 Substituenten, ausgewählt aus der Gruppe, bestehend aus Halogen, Alkylthio-, Alkoxy- oder Alkylgruppen mit 1-4 Kohlenstoffatomen, Hydroxy, Nitro und Trifluormethyl oder A und B sind Phenylreste, in welchen zwei benachbarte C-Atome durch eine Methylendioxy- oder Äthylendioxygruppe verbunden sind,
sowie deren optische Isomeren und die Säureadditionssalze der Verbindungen der allgemeinen Formel I, dadurch gekennzeichnet, dass man in an sich bekannter Weise ein Amin der Formel II

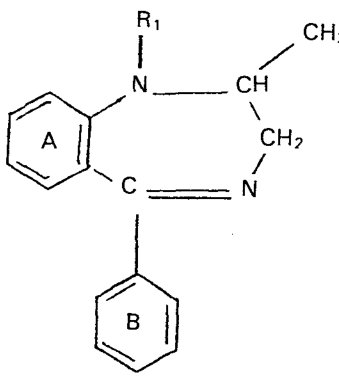

worin A, B, $R_1$ und $R_2$ die oben angegebenen Bedeutungen haben, oder deren Säureadditionssalze mit einer Carbonsäure oder einem reaktiven Carbonsäurederivat der allgemeinen Formel III

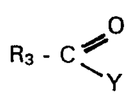

worin $R_3$ die obengenannte Bedeutung hat und Y Hydroxy, Halogen, eine niedermolekulare Alkoxygruppe oder O-CO-Z ist, worin Z die Bedeutung $R_3$ oder niedermolekulare Alkoxygruppe hat, in einem inerten Lösungsmittel bei Temperaturen zwischen $-30°C$ und dem Siedepunkt des eingesetzten Lösungsmittels umsetzt, wobei eine Verbindung der allgemeinen Formel I erhalten wird, in welcher A, B, $R_1$, $R_2$ und $R_3$ die obige Bedeutung haben, $R_2$ mit Bedeutung Wasserstoff ggf. in $R_2$ mit Bedeutung niedermolekulares Alkyl mit bis zu 4 Kohlenstoffatomen überführt, im Ring A in an sich bekannter Weise ggf. Chlor, Brom oder Nitro einführt, ggf. die racemischen Gemische in ihre optischen Isomeren auftrennt und ggf. die gebildete Base in ein Säureadditionssalz überführt oder aus dem Säureadditionssalz die freie Base isoliert.

15. Arzneimittel, enthaltend eine oder mehrere Verbindungen gemäss Anspruch 1 und übliche pharmazeutische Hilfs- und Trägerstoffe.

16. 1-Methyl-2-[(thiophen-3-carbonyl)-aminomethyl]-5-(2-fluorphenyl)-1H-2,3-dihydro-1,4-benzodiazepinhydrochlorid.

17. 8-Fluor-1-methyl-2-[(furan-3-carbonyl)-aminomethyl]-5-phenyl-1H-2,3-dihydro-1,4-benzodiazepinhydrochlorid.

**Patentansprüche** für den Vertragsstaat: AT

1. Verfahren zur Herstellung von 2-Acylaminomethyl-1H-2,3-dihydro-1,4-benzodiazepinen der allgemeinen Formel I

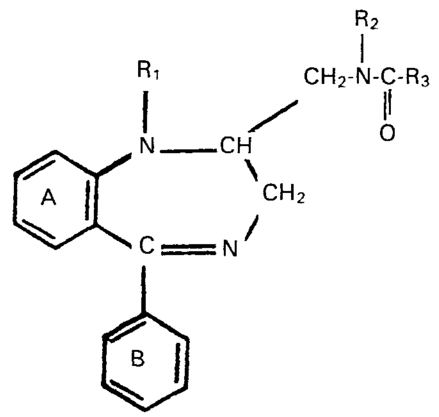

worin $R_1$ ist ein Wasserstoffatom oder eine Alkyl- oder Alkenylgruppe mit bis zu 4 Kohlenstoffatomen oder die Cyclopropylmethylgruppe,
$R_2$ ist ein Wasserstoffatom oder eine niedermolekulare Alkyl- oder Alkenylgruppe mit bis zu 4 Kohlenstoffatomen,
$R_3$ ist eine Gruppe der Formel

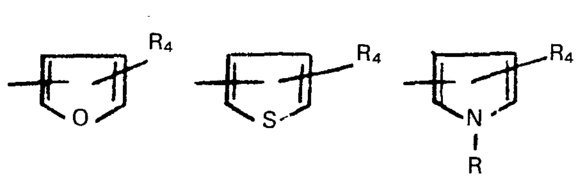

oder

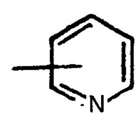

wobei R ist ein Wasserstoffatom oder ein $C_1$-$C_3$-Alkyl,
$R_4$ ist ein Wasserstoffatom, ein Halogenatom, insbesondere Chlor- oder Bromatom, eine Alkyl- oder Alkoxygruppe mit 1-4 Kohlenstoffatomen oder Nitro und
$R_4'$ ist Wasserstoff oder $C_1$-$C_4$-Alkyl,
A und B sind unabhängig voneinander unsubstituiert oder substituiert mit 1 bis 3 Substituenten, ausgewählt aus der Gruppe, bestehend aus Halogen, Alkylthio-, Alkoxy- oder Alkylgruppen mit 1-4 Kohlenstoffatomen, Hydroxy, Nitro und Trifluormethyl oder A und B sind Phenylreste, in welchen zwei benachbarte C-Atome durch eine Methylendioxy- oder Äthylendioxygruppe verbunden sind,
sowie deren optische Isomeren und die Säureadditionssalze der Verbindungen der allgemeinen Formel I, dadurch gekennzeichnet, dass man in an sich bekannter Weise ein Amin der Formel II

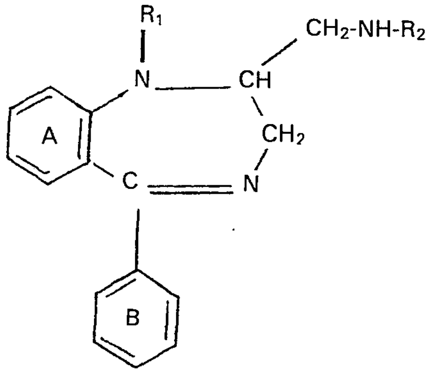

worin A, B, $R_1$ und $R_2$ die oben angegebenen Bedeutungen haben, oder deren Säureadditionssalze mit einer Carbonsäure oder einem reaktiven Carbonsäurederivat der allgemeinen Formel III

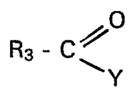

worin $R_3$ die obengenannte Bedeutung hat und Y Hydroxy, Halogen, eine niedermolekulare Alkoxygruppe oder C-CO-Z ist, worin Z die Bedeutung $R_3$ oder niedermolekulare Alkoxygruppe hat, in einem inerten Lösungsmittel bei Temperaturen zwischen −30°C und dem Siedepunkt des eingesetzten Lösungsmittels umsetzt, wobei eine Verbindung der allgemeinen Formel I erhalten wird, in welcher A, B, $R_1$, $R_2$ und $R_3$ die obige Bedeutung haben, $R_2$ mit Bedeutung Wasserstoff ggf. in $R_2$ mit Bedeutung niedemolekulares Alkyl mit bis zu4 Kohlenstoffatomen überführt, im Ring A in ansich bekannter Weise ggf. Chlor, Brom oder Nitro einführt, ggf. die racemischen Gemische in ihre optischen Isomeren auftrennt und ggf. die gebildete Base in ein Säureadditionssalz überführt oder aus dem Säureadditionssalz die freie Base isoliert.

2. Verfahren nach Anspruch 1, dadurch gekennzeichnet, dass als Verbindungen der allgemeinen Formel I 1-Methyl-2-[(thiophen-2-carbonyl)-aminomethyl]-5-phenyl-1H-2,3-dihydro-1,4-benzodiazepine hergestellt werden, worin $R_1$ Methyl bedeutet, $R_2$ Wasserstoff bedeutet, $R_3$ eine 2-Thienylgruppe bedeutet, Ring B unsubstituiert ist und Ring A unsubstituiert ist oder substituiert durch 7-Fluor, 7-Methyl, 7-Isopropyl, 6,8-Dimethyl, 7,8-Dimethyl, 7-Methoxy, 7-n-Propoxy, 7,8-Methylendioxy, 7,8-Äthylendioxy, 7-Trifluormethyl, 7-Nitro, 7,8-Dimethoxy, 6,8-Dimethoxy, 8-Fluor, 8-Chlor, 8-Äthyl, 8-Methylthio, 8-Methoxy oder 8-Äthoxy ist.

3. Verfahren nach Andpruch 1, dadurch gekennzeichnet, dass als Verbindungen der allgemeinen Formel I 1-Methyl-2-[(thiophen-2-carbonyl)-aminomethyl]-5-phenyl-1H-2,3-dihydro-1,4-benzodiazepine hergestellt werden, worin $R_1$ Methyl, $R_2$ Wasserstoff und $R_3$ eine 2-Thienylgruppe bedeuten, Ring A unsubstituiert ist und Ring B unsubstituiert ist oder substituiert ist durch 2-Fluor, 3-Methoxy, 2,6-Dimethoxy oder 3,4-Dimethoxy.

4. Verfahren nach Anspruch 1, dadurch gekennzeichnet, dass als Verbindungen der allgemeinen Formel I 1-Methyl-2-[(thiophen-2-carbonyl)-aminomethyl]-5-phenyl-1H-2,3-dihydro-1,4-benzodiazepine hergestellt werden, worin $R_1$ Methyl, $R_2$ Wasserstoff und $R_3$ eine 2-Thienylgruppe bedeuten, Ring A substituiert ist durch 7-Fluor, 7-Brom, 7-Methyl-, 8-Methoxy oder 7,8-Methylendioxy und Ring B substituiert ist durch 2-Fluor, 3-Fluor, 4-Fluor, 2-Chlor, 2-Brom, 2-Methyl, 2-Trifluormethyl, 3-Trifluormethyl, 4-Trifluormethyl oder 3,4,5-Trimethoxy.

5. Verfahren nach Anspruch 1, dadurch gekennzeichnet, dass als Verbindungen der allgemeinen Formel I 1-Methyl-2-[(thiophen-3-carbonyl)-aminomethyl]-5-phenyl-1H-2,3-dihydro-1,4-benzodiazepine hergestellt werden, worin $R_1$ Methyl, $R_2$ Wasserstoff und $R_3$ eine 3-Thienylgruppe bedeuten, Ring B unsubstituiert ist und Ring A unsubstituiert ist oder substituiert ist durch 7-Methyl, 8-Methyl, 9-Methyl, 8-Äthyl, 7-n-Propyl, 7-Isopropyl, 7-Methoxy, 8-Methoxy, 8-Äthoxy, 8-Methylthio, 6,8-Dimethyl oder 7,8-Dimethyl.

6. Verfahren nach Anspruch 1, dadurch gekennzeichnet, dass als Verbindungen der allgemeinen Formel I 1-Methyl-2-[(thiophen-3-carbonyl)-aminomethyl]-5-phenyl-1H-2,3-dihydro-1,4-benzodiazepine hergestellt werden, worin $R_1$ Methyl und $R_2$ Wasserstoff bedeuten, Ring A unsubstituiert ist oder substituiert ist durch 7-Fluor, 7-Brom, 7-Chlor-8--methoxy, 7-Fluor-8-methoxy, 7-Methyl, 8-Methyl, 9-Methyl, 6-Methoxy, 8-Methoxy, 8-Äthoxy, 8-Isopropoxy, 8-Nitro, 8-Methyl-6-methoxy, 7,8-Methylendioxy, 6,7-Methylendioxy, 6-Hydroxy oder 7,8-Dimethoxy und Ring B unsubstituiert ist oder substituiert ist durch 2-Fluor, 2-Chlor, 3-Methoxy, 4-Methoxy, 3,4-Dimethoxy, 3,4,5-Trimethoxy, 3-Trifluormethyl oder 4-Trifluormethyl und $R_3$ eine durch $R_4$ substituierte 3-Thienylgruppe darstellt, worin $R_4$ Wasserstoff oder 2-Methyl bedeutet.

7. Verfahren gemäss Anspruch 1, dadurch gekennzeichnet, dass als Verbindungen der allgemeinen Formel I 1-Methyl-2-[(furan-2-carbonyl)-aminomethyl]-5-phenyl-1H-2,3-dihydro-1,4-benzodiazepine hergestellt werden, worin $R_1$ Methyl und $R_2$ Wasserstoff bedeuten, Ring A unsubstituiert ist oder substituiert ist durch 7-Fluor, 7-Brom, 7-Methyl, 8-Äthyl, 7-Isopropyl, 8-Methylthio, 6,8-Dimethyl, 7,8-Dimethyl, 7-Methoxy, 8-Methoxy, 6,8-Dimethoxy, 7,8-Dimethoxy, 7-n-Propoxy, 7,8-Methylendioxy, 7-Chlor und Ring B unsubstituiert ist oder substituiert ist durch 2-Chlor, 2-Fluor, 3-Methoxy, 3,4-Dimethoxy, 3,4,5-Trimethoxy und $R_3$ eine durch $R_4$ substituierte 2-Furylgruppe darstellt, worin $R_4$ Wasserstoff, 5-Methyl oder 5-Nitro bedeutet.

8. Verfahren nach Anspruch 1, dadurch gekennzeichnet, dass als Verbindungen der allgemeinen Formel I 1-Methyl-2-[(furan-3-carbonyl)-aminomethyl]-5-phenyl-1H-2,3-dihydro-1,4-benzodiazepine hergestellt werden, worin $R_1$ Methyl und $R_2$ Wasserstoff bedeuten, Ring A unsubstituiert ist oder substituiert ist durch 7-Chlor, 8-Chlor, 7-Fluor, 8-Fluor, 7-Fluor-8-methoxy, 8-Nitro, 7-Methyl-9-chlor, 8-Methylthio, 7-Methyl, 8-Methyl, 8-Methyl-6-methoxy, 8-Äthyl, 7-Isopropyl, 7,8-Dimthyl, 6,8-Dimethyl, 7-Methoxy, 8-Methoxy, 8-Äthoxy, 7-n-Propoxy, 8-Isopropoxy, 7,8-Dimethoxy, 7,8-Methylendioxy, 7,8-Äthylendioxy und Ring B unsubstituiert ist oder substituiert ist durch 2-Fluor, 3-Fluor, 4-Fluor,

2-Brom, 2-Chlor, 2-Methyl, 2-Trifluormethyl, 3-Trifluormethyl, 4-Trifluormethyl, 2,6-Difluor, 3-Trifluormethyl-4-chlor, 2,4-Dichlor, 2-Methoxy, 3-Methoxy, 4-Methoxy, 2,6-Dimethoxy, 3,4-Dimethoxy oder 3,4,5-Trimethoxy und $R_3$ eine durch $R_4$ substituierte 3-Furylgruppe darstellt, worin $R_4$ Wasserstoff, 2-Methyl oder 5-Methyl bedeutet.

9. Verfahren nach Anspruch 1, dadurch gekennzeichnet, dass als Verbindung der allgemeinen Formel I 1-Methyl-2-[(thiophen-3-carbonyl)-aminomethyl]-5-(2-fluorphenyl)-1H-2,3-dihydro-1,4-benzodiazepin hergestellt wird.

10. Verfahren nach Anspruch 1, dadurch gekennzeichnet, dass als Verbindung der allgemeinen Formel I 8-Fluor-1-methyl-2-[(furan-3-carbonyl)-aminomethyl]-5-phenyl-1H-2,3-dihydro-1,4-benzodiazepin hergestellt wird.

**Claims** for the Contracting States:
BE, CH, DE, FR, GB, IT, LI, LU, NL, SE

1. 2-Acylaminomethyl-1H-2,3-dihydro-1,4-benzodiazepines of the general formula I

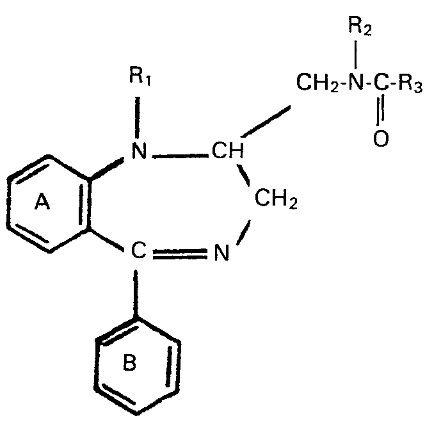

where $R_1$ is a hydrogen atom, or an alkyl or alkenyl radical with up to 4 carbon atoms or the cyclopropylmethyl radical,
$R_2$ is a hydrogen atom or an alkyl or alkenyl radical with up to 4 carbon atoms,
$R_3$ is a radical of the formula

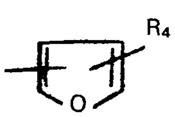

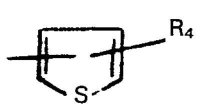

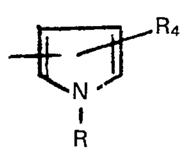

oder 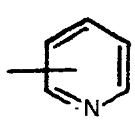

where R is a hydrogen atom or a $C_1$-$C_3$ alkyl
$R_4$ is a hydrogen atom, a halogen atom, especially a chlorine or bromine atom, an alkyl or alkoxy radical with 1 to 4 carbon atoms or nitro and
$R_4'$ is hydrogen or a $C_1$-$C_4$-alkyl
A and B independently of one another are unsubstituted or substituted with 1 to 3 substituents selected from the group consisting of halogen, alkylthio, alkoxy or alkyl radicals with 1 to 4 carbon atoms, hydroxy, nitro and trifluoromethyl, or
A and B are phenyl radicals in which two adjacent C-atoms are linked by a methylenedioxy or ethylenedioxy radical; as well as their optical isomers and the acid addition salts of the compounds of the general formula I.

2. 1-Methyl-2-[(thiophen-2-carbonyl)-aminomethyl]-5-phenyl-1H-2,3-dihydro-1,4-benzodiazepines of the general formula I according to Claim 1,
where $R_1$ stands for methyl,
$R_2$ stands for hydrogen,
$R_3$ stands for a 2-thienyl radical,
ring B is unsubstituted and
ring A is unsubstituted or substituted by 7-fluoro, 7-methyl, 7-isopropyl, 6,8-dimethyl, 7,8-dimethyl, 7-methoxy, 7-n-propoxy, 7,8-methylenedioxy, 7,8-ethylenedioxy, 7-trifluoromethyl, 7-nitro, 7,8-dimethoxy, 6,8-dimethoxy, 8-fluoro, 8-chloro, 8-ethyl, 8-methylthio, 8-methoxy or 8-ethoxy.

3. 1-Methyl-2-[(thiophen-2-carbonyl)-aminomethyl]-5-phenyl-1H-2,3-dihydro-1,4-benzodiazepines of the general formula I according to Claim 1,
where $R_1$ stands for methyl,
$R_2$ stands for hydrogen and
$R_3$ stands for a thienyl radical,
ring A is unsubstituted and
ring B is unsubstituted or is substituted by 2-fluoro, 3-methoxy, 2,6-dimethoxy or 3,4-dimethoxy.

4. 1-Methyl-2-[(thiophen-2-carbonyl)-aminomethyl]-5-phenyl-1H-2,3-dihydro-1,4-benzodiazepines of the general formula I according to Claim 1,
where $R_1$ stands for methyl,
$R_2$ stands for hydrogen and
$R_3$ stands for a thienyl radical,
ring A is substituted by 7-fluoro, 7-bromo, 7-methyl, 8-methoxy or 7,8-methylenedioxy and
ring B is substituted by 2-fluoro, 3-fluoro, 4-fluoro, 2-chloro, 2-bromo, 2-methyl, 2-trifluoromethyl, 3-trifluoromethyl, 4-trifluoromethyl or 3,4,5-trimethoxy.

5. 1-Methyl-2-[(thiophen-2-carbonyl)-aminomethyl]-5-phenyl-1H-2,3-dihydro-1,4-benzodiazepines of the general formula I according to Claim 1,
where $R_1$ stands for methyl,
$R_2$ stands for hydrogen, and
$R_3$ stands for a thienyl radical,
ring A is unsubstituted or is substituted by 7-fluoro, 8-fluoro, 7-methyl, 8-methylthio, 7-methoxy, 8-methoxy, 8-ethoxy, 7-n-propoxy or 7,8-methylenedioxy and
ring B is unsubstituted or is substituted by 2-fluoro, 3-fluoro, 4-fluoro or 3,4,5-trimethoxy, and
$R_3$ is a 2-thienyl radical substituted by $R_4$ where $R_4$ stands for 5-chloro, 4-bromo, 5-bromo, 3-methyl, 4-methyl, 5-methyl, 5-ethyl, 5-methoxy or 5-ethoxy.

6. 1-Methyl-2-[(thiophen-3-carbonyl)-aminomethyl]-5-phenyl-1H-2,3-dihydro-1,4-benzodiazepines of the general formula I according to Claim 1,
where $R_1$ stands for methyl,
$R_2$ stands for hydrogen and
$R_3$ for a 3-thienyl radical,
ring B is unsubstituted and

ring A is unsubstituted or substituted by 7-methyl, 8-methyl, 9-methyl, 8-ethyl, 7-n-propyl, 7-isopropyl, 7-methoxy, 8-methoxy, 8-ethoxy, 8-methylthio, 6,8-dimethyl or 7,8-dimethyl.

7. 1-Methyl-2-[(thiophen-3-carbonyl)-aminomethyl]-5-phenyl-1H-2,3-dihydro-1,4-benzodiazepines of the general formula I according to Claim 1, where $R_1$ stands for methyl, and
$R_2$ stands for hydrogen,
ring A is unsubstituted or is substituted by 7-fluoro, 7-bromo, 7-chloro-8-methoxy, 7-fluoro-8-methoxy, 7-methyl, 8-methyl, 9-methyl, 6-methoxy, 8-methoxy, 8-ethoxy, 8-isopropoxy, 8-nitro, 8-methyl-6-methoxy, 7,8-methylenedioxy, 6,7-methylenedioxy, 6-hydroxy or 7,8-dimethoxy, and
ring B is unsubstituted or is substituted by 2-fluoro, 2-chloro 3-methoxy, 4-methoxy, 3,4-dimethoxy, 3,4,5-trimethoxy, 3-trifluoromethyl or 4-trifluoromethyl and
$R_3$ stands for a 3-thienyl radical substituted by $R_4$ where $R_4$ stands for hydrogen or 2-methyl.

8. 1-Methyl-2-[(furan-2-carbonyl)-aminomethyl]-5-phenyl-1H-2,3-dihydro-1,4-benzodiazepines of the general formula I according to Claim 1, where $R_1$ stands for methyl and
$R_2$ stands for hydrogen,
ring A is unsubstituted or is substituted by 7-fluoro, 7-bromo, 7-methyl, 8-ethyl, 7-isopropyl, 8-methylthio, 6,8-dimethyl, 7,8-dimethyl, 7-methoxy, 8-methoxy, 6,8-dimethoxy, 7,8-dimethoxy, 7-n-propoxy, 7,8-methylenedioxy, 7-chloro and
ring B is unsubstituted or is substituted by 2-chloro, 2-fluoro, 3-methoxy, 3,4-dimethoxy, 3,4,5-trimethoxy and
$R_3$ is a 2-furyl radical substituted by $R_4$, where $R_4$ stands for hydrogen, 5-methyl or 5-nitro.

9. 1-Methyl-2-[(furan-3-carbonyl)-aminomethyl]-5-phenyl-1H-2,3-dihydro-1,4-benzodiazepines of the general formula I according to Claim 1, where $R_1$ stands for methyl and
$R_2$ stands for hydrogen,
ring A is unsubstituted or is substituted by 7-chloro, 8-chloro, 7-fluoro, 8-fluoro, 7-fluoro-8-methoxy, 8-nitro, 7-methyl-9-chloro, 8-methylthio, 7-methyl, 8-methyl, 8-methyl-6-methoxy, 8-ethyl, 7-isopropyl, 7,8-dimethyl, 6,8-dimethyl, 7-methoxy, 8-methoxy, 8-ethoxy, 7-n-propoxy, 8-isopropoxy, 7,8-dimethoxy, 7,8-methylenedioxy, 7,8-ethylenedioxy and
ring B is unsubstituted or is substituted by 2-fluoro, 3-fluoro, 4-fluoro, 2-bromo, 2-chloro, 2-methyl, 2-trifluoromethyl, 3-trifluoromethyl, 4-trifluoromethyl, 2,6-difluoro, 3-trifluoromethyl-4-chloro, 2,4-dichloro, 2-methoxy, 3-methoxy, 4-methoxy, 2,6-dimethoxy, 3,4-dimethoxy or 3,4,5-trimethoxy and
$R_3$ is a 3-furyl radical substituted by $R_4$ where $R_4$ stands for hydrogen, 2-methyl or 5-methyl.

10. 1-Methyl-2-[(pyrrole-2-carbonyl)-aminomethyl]-5-phenyl-1H-2,3-dihydro-1,4-benzodiazepines of the general formula I according to Claim 1, where $R_1$ stands for methyl and
$R_2$ stands for hydrogen and
ring A is unsubstituted or substituted by 8-methyl, 8-methoxy or 7,8-methylenedioxy and
ring B is unsubstituted or substituted by 2-fluoro, 2-bromo, 2-methyl or 4-methoxy and
$R_3$ is a 1H-pyrrole, 1-methylpyrrole or 1,2-dimethylpyrrole ring.

11. 1-Methyl-2-(acylaminomethyl)-5-phenyl-1H-2,3-dihydro-1,4-benzodiazepines of the general formula I according to Claim 1, where $R_1$ stands for methyl and
$R_2$ for hydrogen,
ring A is unsubstituted or substituted by 7-bromo, 7-chloro or 8-methoxy and
ring B is unsubstituted or is substituted by 2-fluoro or 2-chloro, the acyl radical
$R_3CO$ is picolinoyl, nicotinoyl or isonicotinoyl.

12. 2-Acylaminomethyl-1H-2,3-dihydro-1,4-benzodiazepines corresponding to Claim 1, in which $R_1$ is hydrogen, methyl, ethyl, n-propyl, isopropyl, n-butyl or cyclopropylmethyl.

13. 2-Acylaminomethyl-1H-2,3-dihydro-1,4-benzodiazepines corresponding to Claim 1, in which $R_2$ is hydrogen, methyl, ethyl, n-propyl or allyl.

14. Process for the preparation of 2-acylaminomethyl-1H-2,3-dihydro-1,-benzodiazepines of the general formula I according to Claim 1,

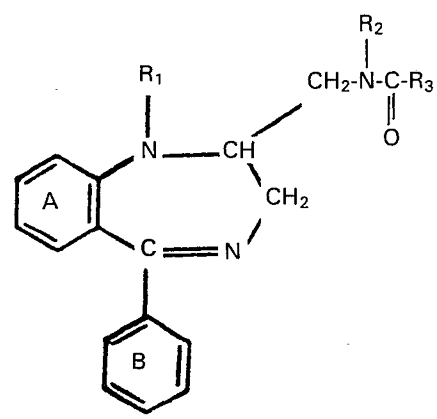

where $R_1$ is a hydrogen atom, an alkyl or alkenyl radical with up to 4 carbon atoms or the cyclopropylmethyl radical,
$R_2$ is a hydrogen atom or a lower molecular alkyl or alkenyl radical with up to 4 carbon atoms,
$R_3$ is a radical of the formula

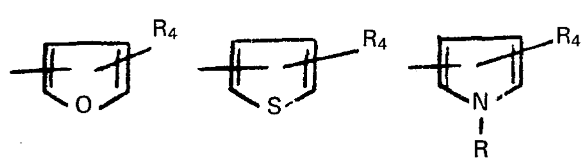

oder 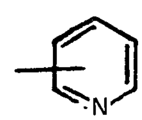

where R is a hydrogen atom or a $C_1$-$C_3$ alkyl,
$R_4$ is a hydrogen atom, a halogen atom, especially a chlorine or bromine atom, an alkyl or alkoxy radical with 1 to 4 carbon atoms or nitro and
$R_4'$ is a hydrogen or $C_1$-$C_4$-alkyl,
A and B independently of one another are unsubstituted or substituted by 1 to 3 substituents selected from the group consisting of halogen alkylthio, alkoxy or alkyl radicals with 1 to 4 carbon atoms, hydroxy, nitro and trifluoromethyl, or

A and B are phenyl radicals in which two adjacent C-atoms are linked by a methylenedioxy or ethylenedioxy radical,

as well as their optical isomers and the acid addition salts of the compounds of the general formula I, characterised in that, in a manner known per se, an amine of the formula II

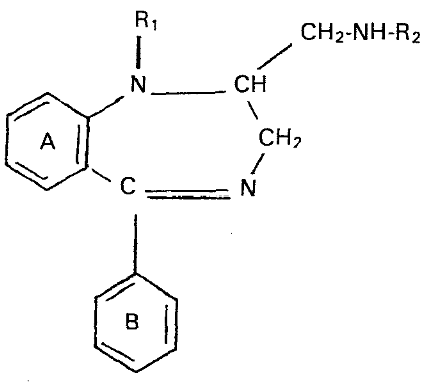

where A, B, $R_1$ and $R_2$ have the meanings defined above, or an acid addition salt thereof, is reacted with a carboxylic acid or a reactive carboxylic acid derivative of the general formula III

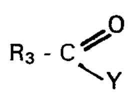

where $R_3$ has the meaning defined above and Y is hydroxy, halogen, a lower molecular alkoxy radical or O-CO-Z radical, where Z stands for $R_3$ or a lower molecular alkoxy radical, in an inert solvent at a temperature between −30°C, and the boiling point of the solvent employed, whereby a compound of the general formula I is obtained in which A, B, $R_1$, $R_2$ and $R_3$ have the above meanings, in the case where $R_2$ stands for hydrogen, it is optionally converted into a lower molecular alkyl radical with up to 4 carbon atoms, if desired, chlorine, bromine or nitro are introduced into ring A in a manner known per se, if desired, the racemic mixture is resolved into its optical isomers; and the base formed is optionally converted into an acid addition salt or the free base is optionally isolated from the acid addition salt.

15. Medicament, containing one or more compounds according to Claim 1, and conventional pharmaceutical auxiliary and carrier materials.

16. 1-Methyl-2-[(thiophen-3-carbonyl)-aminomethyl]-5-(2-fluorophenyl)-1H-2,3-dihydro-1,4-benzodiazepine hydrochloride.

17. 8-Fluoro-1-methyl-2-[(furan-3-carbonyl)-aminomethyl]-5-phenyl-1H-2,3-dihydro-1,4-benzodiazepine hydrochloride.

**Claims** for Contracting State: AT

1. Process for the preparation of 2-acylaminomethyl-1H-2,3-dihydro-1,4-benzodiazepines of the general formula I

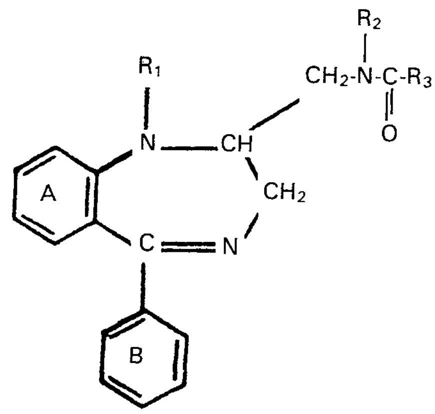

where $R_1$ is a hydrogen atom, an alkyl or alkenyl radical with up to 4 carbon atoms or the cyclopropylmethyl radical,

$R_2$ is a hydrogen atom or a lower molecular alkyl or alkenyl radical with up to 4 carbon atoms,

$R_3$ is a radical of the formula

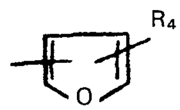 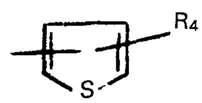 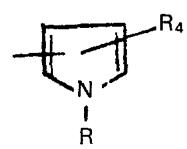

oder 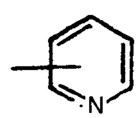

where R is a hydrogen atom or a $C_1$-$C_3$ alkyl,

$R_4$ is a hydrogen atom, a halogen atom, especially a chlorine or bromine atom, an alkyl or alkoxy radical with 1 to 4 carbon atoms or nitro and

$R_4'$ is a hydrogen or $C_1$-$C_4$-alkyl,

A and B independently of one another are unsubstituted or substituted by 1 to 3 substituents selected from the group consisting of halogen alkylthio, alkoxy or alkyl radicals with 1 to 4 carbon atoms, hydroxy, nitro and trifluoromethyl, or

A and B are phenyl radicals in which two adjacent C-atoms are linked by a methylenedioxy or ethylenedioxy radical,

as well as their optical isomers and the acid addition salts of the compounds of the general formula I, characterised in that, in a manner known per se, an amine of the formula II

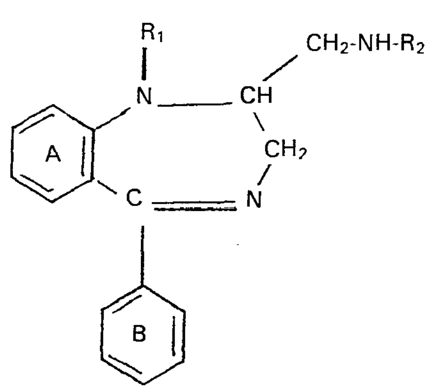

where A, B, $R_1$ and $R_2$ have the meanings defined above, or an acid addition salt thereof, is reacted with a carboxylic acid or a reactive carboxylic acid derivative of the general formula III

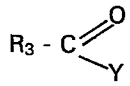

where $R_3$ has the meaning defined above and Y is hydroxy, halogen, a lower molecular alkoxy radical or C-CO-Z radical, where Z stands for $R_3$ or a lower molecular alkoxy radical, in an inert solvent at a temperature between −30°C, and the boiling point of the solvent employed, whereby a compound of the general formula I is obtained in which A, B, $R_1$, $R_2$ and $R_3$ have the above meanings, in the case where $R_2$ stands for hydrogen, it is optionally converted into a lower molecular alkyl radical with up to 4 carbon atoms, if desired, chlorine, bromine or nitro are introduced into ring A in a manner known per se, if desired, the racemic mixture is resolved into its optical isomers; and the base formed is optionally converted into an acid addition salt or the free base is optionally isolated from the acid addition salt.

2. Process according to Claim 1, characterised in that there are prepared as compounds of the general formula I 1-methyl-A-2-[(thiophen-2-carbonyl)-aminomethyl]-5-phenyl-1H-2,3-dihydro-1,4-benzodiazepines,
where $R_1$ stands for methyl,
$R_2$ stands for hydrogen,
$R_3$ stands for a 2-thienyl radical,
ring A is unsubstituted or is substituted by 7-fluoro, 7-methyl, 7-isopropyl, 6,8-dimethyl, 7,8-dimethyl, 7-methoxy, 7-n-propoxy, 7,8-methylenedioxy, 7,8-ethylenedioxy, 7-trifluoromethyl, 7-nitro, 7,8-dimethoxy, 6,8-dimethoxy, 8-fluoro, 8-chloro, 8-ethyl, 8-methylthio, 8-methoxy or 8-ethoxy.

3. Process according to Claim 1, characterised in that there are prepared as compounds of the general formula I 1-methyl-2-[(thiophen-2-carbonyl)-aminomethyl]-5-phenyl-1H-2,3-dihydro-1,4-benzodiazepines,
where $R_1$ stands for methyl,
$R_2$ for hydrogen and
$R_3$ for a 2-thienyl radical,
ring A is unsubstituted and
ring B is unsubstituted or is substituted by 2-fluoro, 3-methoxy, 2,6-dimethoxy or 3,4-dimethoxy.

4. Process according to Claim 1, characterised in that there are prepared as compounds of the general formula I 1-methyl-2-[(thiophen-2-carbonyl)-aminomethyl]-5-phenyl-1H-2,3-dihydro-1,4-benzodiazepines,
where $R_1$ stands for methyl,
$R_2$ for hydrogen and
$R_3$ for a 2-thienyl radical,
ring A is substituted by 7-fluoro, 7-bromo, 7-methyl, 8-methoxy or 7,8-methylenedioxy and
ring B is substituted by 2-fluoro, 3-fluoro, 4-fluoro, 2-chloro, 2-bromo, 2-methyl, 2-trifluoromethyl, 3-trifluoromethyl, 4-trifluoromethyl or 3,4,5-trimethoxy.

5. Process according to Claim 1, characterised in that there are prepared as compounds of the general formula I 1-methyl-2-[(thiophen-3-carbonyl)-aminomethyl]-5-phenyl-1H-2,3-dihydro-1,4-benzodiazepines,
where $R_1$ stands for methyl,
$R_2$ for hydrogen and
$R_3$ for a 3-thienyl radical,
ring B is unsubstituted and
ring A is unsubstituted or is substituted by 7-methyl, 8-methyl, 9-methyl, 8-ethyl, 7-n-propyl, 7-isopropyl, 7-methoxy, 8-methoxy, 8-ethoxy, 8-methylthio, 6,8-dimethyl or 7,8-dimethyl.

6. Process according to Claim 1, characterised in that there are prepared as compounds of the general formula I 1-methyl-2-[(thiophen-3-carbonyl)-aminomethyl]-5-phenyl-1H-2,3-dihydro-1,4-benzodiazepines,
where $R_1$ stands for methyl and
$R_2$ for hydrogen,
ring A is unsubstituted or is substituted by 7-fluoro, 7-bromo, 7-chloro-8-methoxy, 7-fluoro-8-methoxy, 7-methyl, 8-methyl, 9-methyl, 6-methoxy, 8-methoxy, 8-ethoxy, 8-isopropoxy, 8-nitro, 8-methyl-6--methoxy, 7,8-methylenedioxy, 6,7-methylenedioxy, 6-hydroxy or 7,8-dimethoxy and
ring B is unsubstituted or is substituted by 2-fluoro, 2-chloro, 3-methoxy, 4-methoxy, 3,4-dimethoxy, 3,4,5-trimethoxy, 3-trifluoromethyl or 4-trifluoromethyl and $R_3$ is a 3-thienyl radical substituted by $R_4$ where $R_4$ is hydrogen or 2-methyl.

7. Process according to Claim 1, characterised in that there are prepared as compounds of the general formula I 1-methyl-2-[(furan-2-carbonyl)-aminomethyl]-5-phenyl-1H-2,3-dihydro-1,4-benzodiazepines,
where $R_1$ stands for methyl and
$R_2$ for hydrogen,
ring A is unsubstituted or is substituted by 7-fluoro, 7-bromo, 7-methyl, 8-ethyl, 7-isopropyl, 8-methylthio, 6,8-dimethyl, 7,8-dimethyl, 7-methoxy, 8-methoxy, 6,8-dimetoxy, 7,8-dimethoxy, 7-n-propoxy, 7,8-methylenedioxy or 7-chloro and
ring B is unsubstituted or is substituted by 2-chloro, 2-fluoro, 3-methoxy, 3,4-dimethoxy or 3,4,5-trimethoxy and
$R_3$ is a 2-furyl radical substituted by $R_4$ where $R_4$ is hydrogen, 5-methyl or 5-nitro.

8. Process according to Claim 1, characterised in that there are prepared as compounds of the general formula I 1-methyl-2-[(furan-3-carbonyl)-aminomethyl]-5-phenyl-1H-2,3-dihydro-1,4-benzodiazepines,
where $R_1$ stands for methyl and
$R_2$ for hydrogen,
ring A is unsubstituted or substituted by 7-chloro, 8-chloro, 7-fluoro, 8-fluoro, 7-fluoro-8-methoxy, 8-nitro, 7-methyl-9-chloro, 8-methylthio, 7-methyl, 8-methyl, 8-methyl-6-methoxy, 8-ethyl, 7-isopropyl, 7,8-dimethyl, 6,8-dimethyl, 7-methoxy, 8-methoxy, 8-ethoxy, 7-n-propoxy, 8-isopropoxy, 7,8-dimethoxy, 7,8-methylenedioxy, 7,8-ethylenedioxy,
ring B is unsubstituted or substituted by 2-fluoro, 3-fluoro, 4-fluoro, 2-bromo, 2-chloro, 2-methyl, 2-trifluoromethyl, 3-trifluoromethyl, 4-trifluoromethyl, 2,6-difluoro, 3-trifluoromethyl-4-chloro, 2,4-dichloro, 2-methoxy, 3-methoxy, 4-methoxy, 2,6-dimethoxy, 3,4-dimethoxy or 3,4,5,-trimethoxy and $R_3$ is a 3-furyl radical substituted by $R_4$ where $R_4$ is hydrogen, 2-methyl or 5-methyl.

9. Process according to Claim 1, characterised in that there is prepared as compound of the general formula I 1-methyl-2-[(thiophen-3-carbonyl)-aminomethyl]-5-(2-fluorophenyl)-1H-2,3-dihydro-1,4-benzodiazepine.

10. Process according to Claim 1, characterised in that there is prepared as compound of the general formula I 8-fluoro-1-methyl-2-[(furan-3-carbonyl)-aminomethyl]-5-phenyl-1H-2,3-dihydro-1,4-benzodiazepine.

**Revendications** pour les Etats contractants: BE, CH, DE, FR, GB, IT, LI, LU, NL, SE

1. 2-acylaminométhyl-1H-2,3-dihydro-1,4-benzodiazépines de formule générale (I):

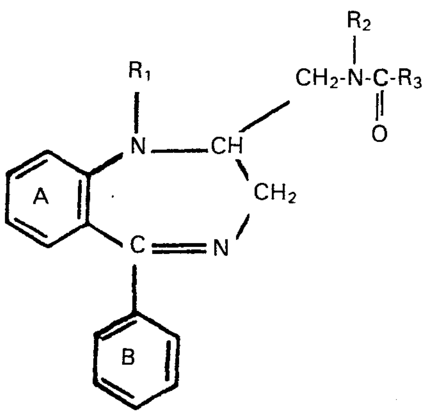

dans laquelle $R_1$ est un atome d'hydrogène ou un groupe alkyle ou alcényle ayant jusqu'à 4 atomes de carbone ou est le groupe cyclopropylméthyle,
$R_2$ est un atome d'hydrogène ou un groupe alkyle ou alcényle ayant jusqu'à 4 atomes de carbone,
$R_3$ est un groupe de formule

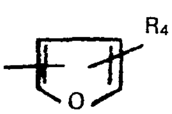 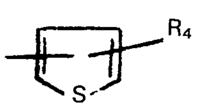 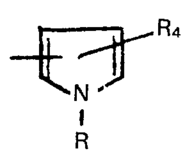

oder 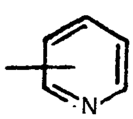

dans lesquelles R est un atome d'hydrogène ou un groupe alkyle ayant 1 à 3 atomes de carbone,
$R_4$ est un atome d'hydrogène, un atome d'halogène notamment un atome de chlore ou de brome, un groupe alkyle ou alcoxy ayant 1 à 4 atomes de carbone ou nitro, et
$R_4'$ est un atome d'hydrogène ou un groupe alkyle ayant 1 à 4 atomes de carbone,
A et B sont, indépendamment l'un de l'autre, non substitués ou substitués par un à trois substituants, choisis dans l'ensemble constitué par les halogènes des groupes alkylthio, alcoxy ou alkyles ayant 1 à 4 atomes de carbone, hydroxyle, nitro et trifluorométhyle, ou
A et B sont des radicaux phényles dans lesquels 2 atomes de carbone voisins sont reliés par un groupe méthylènedioxy ou éthylènedioxy,
ainsi que leurs isomères optiques et les sels d'addition d'acides des composés de formule générale (I).

2. 1-méthyl-2-[(thiophène-2-carbonyl)-aminométhyl]-5-phényl-1H-2,3-dihydro-1,4-benzodiazépines de formule générale (I) selon la revendication 1, dans laquelle $R_1$ est un groupe méthyle, $R_2$ un atome d'hydrogène, $R_3$ un groupe 2-thiényle, le noyau B n'est pas substitué et le noyau A n'est pas substitué ou est substitué par un atome de fluor (position 7), un groupe méthyle (position 7), isopropyle (position 7), deux groupes méthyles (positions 6 et 8), deux groupes méthyles (positions 7 et 8), méthoxy (position 7), n-propoxy (position 7), méthylènedioxy (positions 7 et 8), éthylènedioxy (positions 7 et 8), trifluorométhyle (position 7), nitro (position 7), deux groupes méthoxy (positions 7 et 8), deux groupes méthoxy (position 6 et 8), un atome de fluor (position 8), de chlore (position 8), un groupe éthyle (position 8), méthylthio (position 8), méthoxy (position 8) ou éthoxy (position 8).

3. 1-méthyl-2-[(thiophène-2-carbonyl)-aminométhyl]-5-phényl-1H-2,3-dihydro-1,4-benzodiazépines de formule générale (I) selon la revendication 1, dans laquelle $R_1$ est un groupe méthyle, $R_2$ un atome d'hydrogène et $R_3$ un groupe 2-thiényle, le noyau A n'est pas substitué et le noyau B n'est pas substitué ou est substitué par un atome de fluor (position 2), un groupe méthoxy (position 3), deux groupes méthoxy (positions 2 et 6) ou deux groupes méthoxy (positions 3 et 4).

4. 1-méthyl-2-[(thiophène-2-carbonyl)-aminométhyl]-5-phényl-1H-2,3-dihydro-1,4-benzodiazépines de formule générale (I) selon la revendication 1, dans laquelle $R_1$ est un groupe méthyle, $R_2$ un atome d'hydrogène et $R_3$ un groupe 2-thiényle, le noyau A est substitué par un atome de fluor (position 7), de brome (position 7), un groupe méthyle (position 7), méthoxy (position 8) ou méthylènedioxy (positions 7 et 8) et le noyau B est substitué par un atome de fluor (position 2), de fluor (position 3), de fluor (position 4), de chlore (position 2), de brome (position 2), un groupe méthyle (position 2), trifluorométhyle (position 2), trifluorométhyle (position 3), trifluorométhyle (position 4) ou trois groupes méthoxy (positions 3, 4 et 5).

5. 1-méthyl-2-[(thiophène-2-carbonyl)-aminométhyl]-5-phényl-1H-2,3-dihydro-1,4-benzodiazépines de formule générale (I) selon la revendication 1, dans laquelle $R_1$ est un groupe méthyle et $R_2$ un atome d'hydrogène; le noyau A n'est pas substitué ou est substitué par un atome de fluor (position 7), un atome de fluor (position 8), un groupe méthyle (position 7), méthylthio (position 8), méthoxy (position 7), méthoxy (position 8), éthoxy (position 8), n-propoxy (position 7) ou méthylènedioxy (positions 7 et 8) et le noyau B n'est pas substitué ou est substitué par un atome de fluor (position 2), un atome de

fluor (position 3), un atome de fluor (position 4) ou trois groupes méthoxy (positions 3, 4 et 5), et $R_3$ est un groupe 2-thiényle substitué par $R_4$, où $R_4$ est un atome de chlore (position 5), de brome (position 4), de brome (position 5), un groupe méthyle (position 3), méthyle (position 4), méthyle (position 5), éthyle (position 5), méthoxy (position 5) ou éthoxy (position 5).

6. 1-méthyl-2-[(thiophène-3-carbonyl)-aminométhyl]-5-phényl-1H-2,3-dihydro-1,4-benzodiazépines de formule générale (I) selon la revendication 1, dans laquelle $R_1$ est un groupe méthyle, $R_2$ un atome d'hydrogène et $R_3$ un groupe 3-thiényle, le noyau B n'est pas substitué et le noyau A n'est pas substitué ou est substitué par un groupe méthyle (position 7), méthyle (position 8), méthyle (position 9), éthyle (position 8), n-propyle (position 7), isopropyle (position 7), méthoxy (position 7), méthoxy (position 8), éthoxy (position 8), méthylthio (position 8), deux groupes méthyles (positions 6 et 8) ou deux groupes méthyles (positions 7 et 8).

7. 1-méthyl-2-[(thiophène-3-carbonyl)-aminométhyl]-5-phényl-1H-2,3-dihydro-1,4-benzodiazépines de formule générale (I) selon la revendication 1, dans laquelle $R_1$ est un groupe méthyle et $R_2$ un atome d'hydrogène, le noyau A n'est pas substitué ou est substitué par un atome de fluor (position 7), de brome (position 7), de chlore (position 7) et un groupe méthoxy (position 8), un atome de fluor (position 7) et un groupe méthoxy (position 8), un groupe méthyle (position 7), un groupe méthyle (position 8), méthyle (position 9), méthoxy (position 6), méthoxy (position 8), éthoxy (position 8), isopropoxy (position 8), nitro (position 8), méthyle (position 8) et méthoxy (position 6), méthylènedioxy (positions 7 et 8), méthylènedioxy (positions 6 et 7), hydroxyle (position 6) ou deux groupes méthoxy (positions 7 et 8) et le noyau B n'est pas substitué ou est substitué par un atome de fluor (position 2), de chlore (position 2), un groupe méthoxy (position 3), méthoxy (position 4), deux groupes méthoxy (positions 3 et 4), trois groupes méthoxy (positions 3, 4 et 5), trifluorométhyle (position 3) ou trifluorométhyle (position 4), et $R_3$ est un groupe 3-thiényle substitué par $R_4$, où $R_4$ est un atome d'hydrogène ou un groupe méthyle (position 2).

8. 1-méthyl-2-[(furanne-2-carbonyl)-aminométhyl]-5-phényl-1H-2,3-dihydro-1,4-benzodiazépines de formule générale (I) selon la revendication 1, dans laquelle $R_1$ est un groupe méthyle et $R_2$ un atome d'hydrogène, le noyau A n'est pas substitué ou est substitué par un atome de fluor (position 7), de brome (position 7), un groupe méthyle (position 7), éthyle (position 8), isopropyle (position 7), méthylthio (position 8), deux groupes méthyles (positions 6 et 8), deux groupes méthyles (positions 7 et 8), un groupe méthoxy (position 7), un groupe méthoxy (position 8), deux groupes méthoxy (positions 6 et 8), deux groupes méthoxy (positions 7 et 8), un groupe n-propoxy (position 7), un groupe méthylènedioxy (positions 7 et 8), un atome de chlore (position 7) et le noyau B n'est pas substitué ou est substitué par un atome de chlore (position 2), de fluor (position 2), un groupe méthoxy (position 3), deux groupes méthoxy (positions 3 et 4), ou trois groupes méthoxy (positions 3, 4 et 5) et $R_3$ est un groupe 2-furyle substitué par $R_4$, où $R_4$ est un atome d'hydrogène, un groupe méthyle (position 5) ou nitro (position 5).

9. 1-méthyl-2-[(furanne-3-carbonyl)-aminométhyl]-5-phényl-1H-2,3-dihydro-1,4-benzodiazépines de formule générale (I) selon la revendication 1, dans laquelle $R_1$ est un groupe méthyle et $R_2$ un atome d'hydrogène, le noyau A n'est pas substitué ou est substitué par un atome de chlore (position 7), un atome de chlore (position 8), de fluor (position 7), de fluor (position 8), un atome de fluor (position 7) et un groupe méthoxy (position 8), un groupe nitro (position 8), un groupe méthyle (position 7) et un atome de chlore (position 9), un groupe méthylthio (position 8), un groupe méthyle (position 7), un groupe méthyle (position 8), un groupe méthyle (position 8) et méthoxy (position 6), un groupe éthyle (position 8), isopropyle (position 7), deux groupes méthyles (positions 7 et 8), deux groupes méthyles (positions 6 et 8), un groupe méthoxy (position 7), méthoxy (position 8), éthoxy (position 8), n-propoxy (position 7), isopropoxy (position 8), deux groupes méthoxy (positions 7 et 8), un groupe méthylènedioxy (positions 7 et 8), un groupe éthylènedioxy (positions 7 et 8), et le noyau B n'est pas substitué ou est substitué par un atome de fluor (position 2), un atome de fluor (position 3), un atome de fluor (position 4), un atome de brome (position 2), de chlore (position 2), un groupe méthyle (position 2), trifluorométhyle (position 2), trifluorométhyle (position 3), trifluorométhyle (position 4), deux atomes de fluor (positions 2 et 6), un groupe trifluorométhyle (position 3) et un atome de chlore (position 4), deux atomes de chlore (positions 2 et 4), un groupe méthoxy (position 2), méthoxy (position 3), méthoxy (position 4), deux groupes méthoxy (positions 2 et 6), deux groupes méthoxy (position 3 et 4) ou trois groupes méthoxy (positions 3, 4 et 5), et $R_3$ est un groupe 3-furyle substitué par $R_4$, où $R_4$ est un atome d'hydrogène, un groupe méthyle (position 2) ou méthyle (position 5).

10. 1-méthyl-2-[(pyrrole-2-carbonyl)-aminométhyl]-5-phényl-1H-2,3-dihydro-1,4-benzodiazépines de formule générale (I) selon la revendication 1, dans laquelle $R_1$ est un groupe méthyle et $R_2$ un atome d'hydrogène, et le noyau A n'est pas substitué ou est substitué par un groupe méthyle (position 8), méthoxy (position 8) ou méthylènedioxy (positions 7 et 8) et le noyau B n'est pas substitué ou est substitué par un atome de fluor (position 2), de brome (position 2), un groupe méthyle (position 2) ou méthoxy (position 4), et $R_3$ est un noyau 1H-pyrrole, 1-méthylpyrrole ou 1,2-diméthylpyrrole.

11. 1-méthyl-2-(acylaminométhyl)-5-phényl-1H-2,3-dihydro-1,4-benzodiazépines de formule générale (I) selon la revendication 1, dans laquelle $R_1$ est un groupe méthyle et $R_2$ un atome d'hydrogène, le noyau A n'est pas substitué ou est substitué par un atome de brome (position 7), ou de chlore (position 7) ou un groupe méthoxy (position 8) et le noyau B n'est pas substitué ou est substitué par un atome de fluor (position 2) ou de chlore (position 2), et le groupe acyle $R_3CO$ représente un radical picolinoyle, nicotinoyle ou isonicotinoyle.

12. 2-acylaminométhyl-1H-2,3-dihydro-1,4--benzodiazépines selon la revendication 1, dans laquelle $R_1$ est un atome d'hydrogène ou un groupe méthyle, éthyle, n-propyle, isopropyle, n-butyle ou cyclopropylméthyle.

13. 2-acylaminométhyl-1H-2,3-dihydro-1,4--benzodiazépines selon la revendication 1, dans laquelle $R_2$ est un atome d'hydrogène ou un groupe méthyle, éthyle, n-propyle ou allyle.

14. Procédé de préparation de 2-acylaminométhyl-1H-2,3-dihydro-1,4-benzodiazépines de formule générale (I) selon la revendication 1:

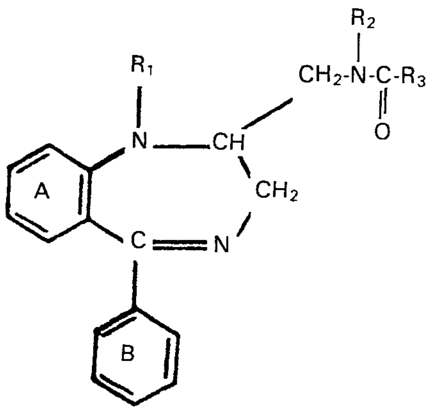

dans laquelle $R_1$ est un atome d'hydrogène ou un groupe alkyle ou alcényle ayant jusqu'à 4 atomes de carbone ou est le groupe cyclopropylméthyle,
$R_2$ est un atome d'hydrogène ou un groupe alkyle ou alcényle à bas poids moléculaire ayant jusqu'à 4 atomes de carbone,
$R_3$ est un groupe de formule:

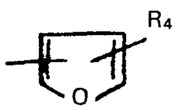 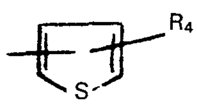 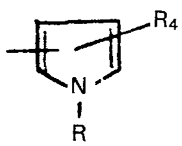

oder 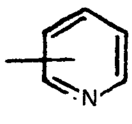

où R est un atome d'hydrogène ou un groupe alkyle en $C_1$-$C_3$,
$R_4$ est un atome d'hydrogène, un atome d'halogène, notamment un atome de chlore ou de brome, un groupe alkyle ou alcoxy ayant 1 à 4 atomes de carbone ou nitro, et
$R_4'$ est un atome d'hydrogène ou un groupe alkyle en $C_1$-$C_4$,
A Et B sont, indépendamment l'un de l'autre, non substitués ou substitués par un à trois substituants, choisis dans l'ensemble constitué par des halogènes, des groupes alkylthio, alcoxy ou alkyles ayant 1 à 4 atomes de carbone, hydroxyle, nitro et trifluorométhyle, ou
A et B sont des radicaux phényles, dans lesquels 2 atomes de carbone voisins sont reliés par un groupe méthylènedioxy ou éthylènedioxy,
ainsi que de leurs isomères optiques et des sels d'addition d'acides des composés de formule générale (I), procédé caractérisé en ce qu'on fait réagir de façon connue en soi une amine de formule (II):

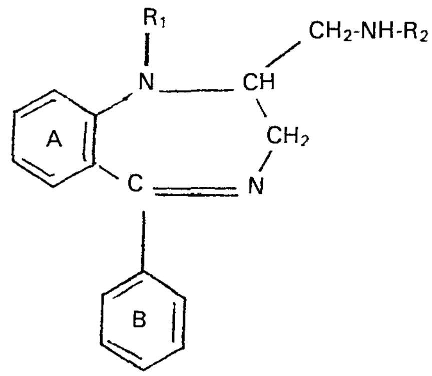

dans laquelle A, B, $R_1$ et $R_2$ ont les sens indiqués ci-dessus ou leurs sels d'addition d'acides avec un acide carboxylique ou un dérivé réactif d'acide carboxylique de formule générale (III):

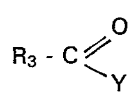

(dans laquelle $R_3$ a le sens indiqué ci-dessus et Y est un groupe hydroxyle, un atome d'halogène, un groupe alcoxy à bas poids moléculaire ou O-CO-Z, où Z a le sens $R_3$ ou est un groupe alcoxy à bas poids moléculaire) dans un solvant inerte à des températures comprises entre $-30\,°C$ et le point d'ébullition du solvant utilisé, de sorte que l'on obtient un composé de formule générale (I), dans laquelle A, B, $R_1$, $R_2$ et $R_3$ ont le sens ci-dessus; on transforme éventuellement $R_2$ représentant un atome d'hydrogène en $R_2$ représentant un groupe alkyle à bas poids moléculaire ayant jusqu'à 4 atomes de carbone; on introduit éventuellement et de façon connue en soi du chlore, du brome ou un groupe nitro dans le noyau A, on sépare éventuellement les mélanges racémiques en leurs isomères optiques et l'on transforme éventuellement la base formée en un sel d'addition d'acide ou l'on isole la base libre à partir du sel d'addition d'acide.

15. Médicament, contenant un ou plusieurs composés selon la revendication 1, et des adjuvants et excipients pharmaceutiques usuels.

16. 1-méthyl-2-[(thiophène-3-carbonyl]-aminométhyl]-5-(2-fluorophényl)-1H-2,3-dihydro-1,4--benzodiazépine hydrochloride.

17. 8-fluoro-1-méthyl-2-[(furanne-3-carbonyl)-aminométhyl]-5-phényl-1H-2,3-dihydro-1,4-benzodiazépine hydrochloride.

**Revendications** pour l'Etat contractant: AT

1. Procédé de préparation de 2-acylaminométhyl-1H-2,3-dihydro-1,4-benzodiazépines de formule générale (I):

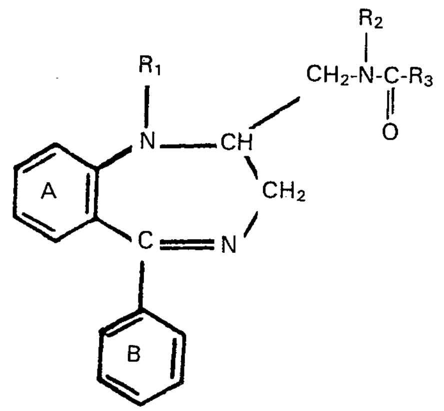

dans laquelle $R_1$ est un atome d'hydrogène ou un groupe alkyle ou alcényle ayant jusqu'à 4 atomes de carbone ou est le groupe cyclopropylméthyle,
$R_2$ est un atome d'hydrogène ou un groupe alkyle ou alcényle à bas poids moléculaire ayant jusqu'à 4 atomes de carbone,
$R_3$ est un groupe de formule:

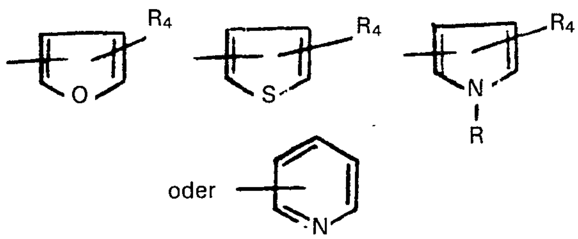
oder

où R est un atome d'hydrogène ou un groupe alkyle en $C_1$-$C_3$,
$R_4$ est un atome d'hydrogène, un atome d'halogène, notamment un atome de chlore ou de brome, un groupe alkyle ou alcoxy ayant 1 à 4 atomes de carbone ou nitro, et
$R_4'$ est un atome d'hydrogène ou un groupe alkyle en $C_1$-$C_4$,
A et B sont, indépendamment l'un de l'autre, non substitués ou substitués par un à trois substituants choisis dans l'ensemble consistant en des halogènes, des groupes alkylthio, alcoxy ou alkyles ayant 1 à 4 atomes de carbone, hydroxyle, nitro et trifluorométhyle, ou
A et B sont des radicaux phényles, dans lesquels 2 atomes de carbone voisins sont reliés par un groupe méthylènedioxy ou éthylènedioxy,
ainsi que leurs isomères optiques et les sels d'addition d'acides des composés de formule générale (I), procédé caractérisé en ce qu'on fait réagir de façon connue en soi une amine de formule (II):

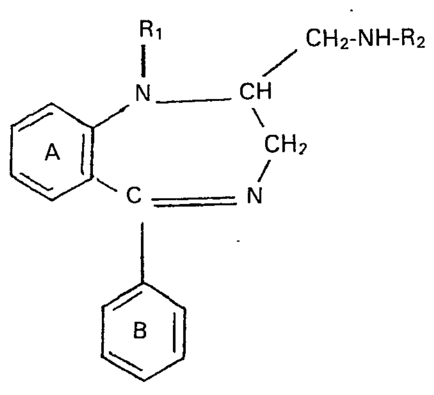

dans laquelle A, B, $R_1$ et $R_2$ ont les sens indiqués ci-dessus ou leurs sels d'addition d'acides avec un acide carboxylique ou avec un dérivé réactif d'acide carboxylique de formule générale (III):

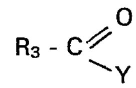

(dans laquelle $R_3$ a le sens précité et Y est un groupe hydroxyle, un atome d'halogène, un groupe à bas poids moléculaire ou O-CO-Z, où Z a le sens $R_3$ ou est un groupe alcoxy à bas poids moléculaire) dans un solvant inerte à des températures comprises entre $-30°C$ et le point d'ébullition du solvant utilisé, de sorte que l'on obtient un composé de formule générale (I), dans laquelle A, B, $R_1$, $R_2$ et $R_3$ ont le sens ci-dessus, et l'on transforme éventuellement $R_2$, lorsqu'il représente un atome d'hydrogène, en $R_2$ représentant un groupe alkyle à bas poids moléculaire ayant jusqu'à 4 atomes de carbone; dans le noyau A, on introduit éventuellement, de façon connue en soi, un atome de chlore ou de brome ou un groupe nitro; on sépare éventuellement les mélanges racémiques en leurs isomères optiques et l'on transforme éventuellement la base formée en un sel d'addition d'acide ou l'on isole la base libre à partir du sel d'addition d'acide.

2. Procédé selon la revendication 1, caractérisé en ce qu'on prépare comme composés de formule générale (I) des 1-méthyl-2-[(thiophène,2-carbonyl)-aminométhyl]-5-phényl-1H-2,3-dihydro-1,4-benzodiazépines, dans lesquelles $R_1$ est un groupe méthyle, $R_2$ un atome d'hydrogène, $R_3$ un groupe thiényle en position 2, le noyau B n'est pas substitué et le noyau A n'est pas substitué ou est substitué par un atome de fluor (position 7), un groupe méthyle (position 7), isopropyle (position 7), deux groupes méthyles (positions 6 et 8), deux groupes méthyles (positions 7 et 8), un groupe méthoxy (position 7), n-propoxy (position 7), méthylènedioxy (positions 7 et 8), éthylènedioxy (positions 7 et 8), trifluorométhyle (position 7), nitro (position 7), deux groupes méthoxy (positions 7 et 8), deux groupes méthoxy (positions 6 et 8), un atome de fluor (position 8), un atome de chlore (position 8), un groupe éthyle (position 8), méthylthio (position 8), méthoxy (position 8) ou éthoxy (position 8).

3. Procédé selon la revendication 1, caractérisé en ce qu'on prépare comme composés de formule générale (I) des 1-méthyl-2-[(thiophène-2-carbonyl)-aminométhyl]-5-phényl-1H-2,3-dihydro-1,4-benzodiazépines, dans lesquelles $R_1$ est un groupe méthyle, $R_2$ un atome d'hydrogène et $R_3$ un groupe thiényle (position 2), le noyau A n'est pas substitué et le noyau B n'est pas substitué ou est substitué par un atome de fluor (position 2), un groupe méthoxy (position 3), deux groupes méthoxy (positions 2 et 6) ou deux groupes méthoxy (positions 3 et 4).

4. Procédé selon la revendication 1, caractérisé en ce qu'on prépare comme composés de formule générale (I) des 1-méthyl-2-[(thiophène-2-carbonyl)-aminométhyl]-5-phényl-1H-2,3-dihydro-1,4-benzodiazépines, dans lesquelles $R_1$ est un groupe méthyle, $R_2$ un atome d'hydrogène et $R_3$ un groupe

thiényle (position 2), le noyau A est substitué par un atome de fluor (position 7), de brome (position 7), un groupe méthyle (position 7), méthoxy (position 8) ou méthylènedioxy (positions 7 et 8) et le noyau B est substitué par un atome de fluor (position 2), un atome de fluor (position 3), un atome de fluor (position 4), un atome de chlore (position 2), un atome de brome (position 2), un groupe méthyle (position 2), trifluorométhyle (position 2), trifluorométhyle (position 3), trifluorométhyle (position 4) ou trois groupes méthoxy (positions 3, 4 et 5).

5. Procédé selon la revendication 1, caractérisé en ce qu'on prépare comme composés de formule générale (I) des 1-méthyl-2-[(thiophène-3-carbonyl)-aminométhyl]-5-phényl-1H-2,3-dihydro-1,4-benzodiazépines dans lesquelles $R_1$ est un groupe méthyle, $R_2$ un atome d'hydrogène et $R_3$ un groupe thiényle (position 3), le noyau B n'est pas substitué et le noyau A n'est pas substitué ou est substitué par un groupe méthyle (position 7), un groupe méthyle (position 8), un groupe méthyle (position 9), un groupe éthyle (position 8), n-propyle (position 7), isopropyle (position 7), méthoxy (position 7), méthoxy (position 8), éthoxy (position 8), méthylthio (position 8), deux groupes méthyles (positions 6 et 8) ou deux groupes méthyles (positions 7 et 8).

6. Procédé selon la revendication 1, caractérisé en ce qu'on prépare comme composés de formule générale (I) des 1-méthyl-2-[(thiophène-3-carbonyl)-aminométhyl]-5-phényl-1H-2,3-dihydro-1,4-benzodiazépines, dans lesquelles $R_1$ est un groupe méthyle et $R_2$ un atome d'hydrogène, le noyau A n'est pas substitué ou est substitué par un atome de fluor (position 7), un atome de brome (position 7), un atome de chlore (position 7) et un groupe méthoxy (position 8), un atome de fluor (position 7) et un groupe méthoxy (position 8), un groupe méthyle (position 7), méthyle (position 8), méthyle (position 9), méthoxy (position 6), méthoxy (position 8), éthoxy (position 8), isopropoxy (position 8), nitro (position 8), un groupe méthyle (position 8) et méthoxy (position 6), un groupe méthylènedioxy (positions 7 et 8), un groupe méthylènedioxy (positions 6 et 7), hydroxyle (position 6) ou deux groupes méthoxy (positions 7 et 8), et le noyau B n'est pas substitué ou est substitué par un atome de fluor (position 2), un atome de chlore (position 2), un groupe méthoxy (position 3), méthoxy (position 4), deux groupes méthoxy (positions 3 et 4), trois groupes méthoxy (positions 3, 4 et 5), un groupe trifluorométhyle (position 3) ou trifluorométhyle (position 4), et $R_3$ est un groupe 3-thiényle substitué par $R_4$, où $R_4$ est un atome d'hydrogène ou un groupe méthyle (position 2).

7. Procédé selon la revendication 1, caractérisé en ce qu'on prépare comme composés de formule générale (I) des 1-méthyl-2-[(furanne-2-carbonyl)-aminométhyl]-5-phényl-1H-2,3-dihydro-1,4-benzodiazépines, dans lesquelles $R_1$ est un groupe méthyle et $R_2$ un atome d'hydrogène, le noyau A n'est pas substitué ou est substitué par un atome de fluor (position 7), de brome (position 7), un groupe méthyle (position 7), éthyle (position 8), isopropyle (position 7), méthylthio (position 8), deux groupes méthyles (positions 6 et 8), deux groupes méthyles (positions 7 et 8), un groupe méthoxy (position 7), méthoxy (position 8), deux groupes méthoxy (positions 6 et 8), deux groupes méthoxy (positions 7 et 8), un groupe n-propoxy (position 7), méthylènedioxy (positions 7 et 8) un atome de chlore (position 7) et le noyau B n'est pas substitué ou est substitué par un atome de chlore (position 2), un atome de fluor (position 2), un groupe méthoxy (position 3), deux groupes méthoxy (positions 3 et 4), trois groupes méthoxy (positions 3, 4 et 5), et $R_3$ représente un groupe 2-furyle substitué par $R_4$, où $R_4$ est un atome d'hydrogène, un groupe méthyle (position 5) ou nitro (position 5).

8. Procédé selon la revendication 1, caractérisé en ce qu'on prépare, comme composés de formule générale (I) des 1-méthyl-2-[(furanne-3-carbonyl)-aminométhyl]-5-phényl-1H-2,3-dihydro-1,4-benzodiazépines, dans lesquelles $R_1$ est un groupe méthyle et $R_2$ un atome d'hydrogène, le noyau A n'est pas substitué ou est substitué par un atome de chlore (position 7), un atome de chlore (position 8), un atome de fluor (position 7), un atome de fluor (position 8), un atome de fluor (position 7) et un groupe méthoxy (position 8), un groupe nitro (position 8), un groupe méthyle (position 7) et un atome de chlore (position 9), un groupe méthylthio (position 8), méthyle (position 7), méthyle (position 8), un groupe méthyle (position 8) et un groupe méthoxy (position 6), un groupe éthyle (position 8), isopropyle (position 7), deux groupes méthyles (positions 7 et 8), deux groupes méthyles (positions 6 et 8), un groupe méthoxy (position 7), méthoxy (position 8), éthoxy (position 8), n-propoxy (position 7), isopropoxy (position 8), deux groupes méthoxy (positions 7 et 8), un groupe méthylènedioxy (positions 7 et 8), éthylènedioxy (positions 7 et 8), et le noyau B n'est pas substitué ou est substitué par un atome de fluor (position 2), un atome de fluor (position 3), un atome de fluor (position 4), un atome de brome (position 2), un atome de chlore (position 2), un groupe méthyle (position 2), trifluorométhyle (position 2), trifluorométhyle (position 3), trifluorométhyle (position 4), deux atomes de fluor (positions 2 et 6), un groupe trifluorométhyle (position 3) et un atome de chlore (position 4), deux atomes de chlore (position 2 et 4), un groupe méthoxy (position 2), méthoxy (position 3), méthoxy (position 4), deux groupes méthoxy (positions 2 et 6), deux groupes méthoxy (positions 3 et 4) ou trois groupes méthoxy (positions 3, 4 et 5), et $R_3$ est un groupe 3-furyle substitué par $R_4$, où $R_4$ est un atome d'hydrogène, un groupe méthyle (position 2) ou méthyle (position 5).

9. Procédé selon la revendication 1, caractérisé en ce qu'on prépare comme composé de formule générale (I) la 1-méthyl-2-[(thiophène-3-carbonyl)-aminométhyl]-5-(2-fluorophényl)-1H-2,3-dihydro-1,4-benzodiazépine.

10. Procédé selon la revendication 1, caractérisé en ce qu'on prépare comme composé de formule générale (I) la 8-fluoro-1-méthyl-2-[(furanne-3-carbonyl)-aminométhyl]-5-phényl-1H-2,3-dihydro-1,4-benzodiazépine.